# EUROPEAN PATENT SPECIFICATION

(11) **EP 4 355 793 B1**
(45) Date of publication and mention of the grant of the patent: **30.10.2024**
(21) Application number: 22733955.3
(22) Date of filing: 15.06.2022
(51) Int. Cl.: C08F 8/30, C08F 220/54, C08G 81/02, C08G 83/00, C08G 85/00, C12N 5/00, C12N 5/074

(54) **THERMORESPONSIVE HYDROGELS AS MICRONICHES FOR THE GROWTH AND CONTROLLED RELEASE OF PLURIPOTENT STEM CELLS AND PREPARATION METHOD THEREOF**
THERMOREAKTIVE HYDROGELE ALS MIKRONISCHEN FÜR DAS WACHSTUM UND KONTROLLIERTE FREISETZUNG PLURIPOTENTER STAMMZELLEN UND VERFAHREN ZU IHRER HERSTELLUNG
HYDROGELS THERMOSENSIBLES EN TANT QUE MICRONICHES POUR LA CROISSANCE ET LA LIBÉRATION CONTRÔLÉE DE CELLULES SOUCHES PLURIPOTENTES ET LEUR PROCÉDÉ DE PRÉPARATION

(30) Priority: 17.06.2021 EP 21180180
(43) Date of publication of application: 24.04.2024
(73) Proprietor: Freie Universität Berlin, 14195 Berlin (DE)
(72) Inventor: HAAG, Rainer, 12209 Berlin (DE); LI, Wenzhong, 14513 Teltow (DE); LIANG, Wanjun, 12203 Berlin (DE)
(74) Representative: Maikowski & Ninnemann Patentanwälte Partnerschaft mbB
(86) International application number: PCT/EP2022/066399
(87) International publication number: WO 2022/263560

(56) References cited:
- WO-A2-2004/085712
- ENRIQUE LALLANA OZORES ET AL: "Click Chemistry with Polymers, Dendrimers, and Hydrogels for Drug Delivery Methods for the simplification of the NMR of complex mixtures and pseudo separations View project Dendrimers for biomedical applications View project", 25 January 2012 (2012-01-25), XP055598051, Retrieved from the Internet <URL:file:///C:/Users/HS51195/Downloads/art3A10.10072Fs11095-012-0683-y.pdf> [retrieved on 20190619], DOI: 10.1007/s11095-012-0683-y·Source:
- MARCELO CALDERÓN ET AL: "Dendritic Polyglycerols for Biomedical Applications", ADVANCED MATERIALS, vol. 22, no. 2, 7 October 2009 (2009-10-07), pages 190 - 218, XP055015111, ISSN: 0935-9648, DOI: 10.1002/adma.200902144
- LIANG WANJUN ET AL: "Thermoresponsive Hydrogels as Microniches for Growth and Controlled Release of Induced Pluripotent Stem Cells", vol. 31, no. 40, 10 October 2021 (2021-10-10), pages 2010630, XP009531940, ISSN: 1057-9257, Retrieved from the Internet <URL:https://onlinelibrary.wiley.com/doi/full/10.1002/adfm.202010630> [retrieved on 20210710], DOI: 10.1002/ADFM.202010630

## Description

The instant invention relates to a thermoresponsive hydrogel according to the preamble of claim 1, to the use of such a thermoresponsive hydrogel according to the preamble of claim 6, to a method for cultivating cells in vitro according to the preamble of claim 8, and to a method for manufacturing such a thermoresponsive hydrogel according to the preamble of claim 15.

The discovery of the induced pluripotent stem cells (iPSCs) has opened up unprecedented opportunities in the area of clinical therapy, especially for regenerative medicine.' Since iPSCs can be cultured from the patient's cells, transplantation therapies based on these patient-specific cell lines could largely eliminate the immunological rejection^{1b, 1d} and meanwhile avoid prominent ethical problems.^{1b, 2} They are serving as a powerful tool in numerous applications.³

However, it should be noted that large amounts of cells with high quality are needed during most of the applications. ⁴ For example, at least 10⁸ -10¹⁰ cells are required per patient in the most current cell therapy protocols and those under development.⁵ The therapeutic use of the iPSCs cells, therefore, greatly necessitates their efficient expansion in large scale under safety-reliable and well-defined culture conditions.

Actually, since the discovery of iPSCs, numerous approaches and culture protocols have been explored to establish scaffolds⁶ or niches⁷ equipped with a certain environment for their survival and expansion in ways with high maneuverability.⁸ Currently, mainstream iPSCs cultures are using laminin as a substrate for cell attachment while growing them on the dish.⁹

However, this substrate is typically 2-dimensional (2D) in nature with a limited ability to recapitulate *in* vivo-like 3D microenvironment.¹⁰ Furthermore, various recent reports suggest that cells cultured on 2D substrates differ greatly from those grown *in vivo.*¹¹ Additional drawbacks include inherent heterogeneity, limited scalability and reproducibility.^{4b, 8b}

The most widely used attempt to move cultures from 2D to 3D is to embed cells into Matrigel.¹² Matrigel is useful for research purposes, while its semi-chemically defined, xenogeneic substrate property, together with the source of variability and xenogeneic contamination make it incompatible for the generation of clinical grade iPSCs.¹³

Thus developing alternative materials for productive and safe 3D culture environment are strongly desired. Advances in materials chemistry,^{6, 8c, 14, 14d} fabricating and processing technologies,^{12, 15} and developmental biology^{13a, 16} have to be coordinately applied in the design of new 3D cell culture matrices that better represent the geometry, chemistry, and signaling environment of the natural extracellular matrix.

Extracellular matrix (ECM) is the natural environment for the cell growth and survival. Thus, building a 3D microenvironment by mimicking the properties of the ECM is a promising strategy.^{10, 16b} Recently, synthetic materials inspired from the construction and role of native ECMs in cell accommodation were produced for the cell culture applications. Hydrogels based on biocompatible polymers were produced which incorporated biological cues to define an artificial milieu with complex interactions that regulate and foster stem cells similar to the events occurring in a natural cellular microenvironment.¹⁷

For example, Lutolf and coworkers reported adhesion peptide functionalized enzymatically crosslinked PEG-based hydrogels by chemical gelation for the design of 3D microenvironment to mimic the biochemical features of the native ECMs.^{16b} After modulating micro-environmental stiffness, degradability, and biochemical composition, this synthetic 3D 'reprogramming niche' was explored for somatic cell reprogramming and iPSC generation. These synthetic 3D cell culture matrices better represented the geometry, chemistry, and signaling environment of the natural extracellular matrix. However, these synthetic 3D systems might not be suitable for the iPSC culture because enzymatic degradation will inevitably be harmful to the proliferated cells.

The Haag group has previously reported a microgel construction kit and successfully realized the pH-controlled release of living cells from the hydrogel by benzacetal bond hydrolysis.¹⁸ However, iPSCs are too sensitive to survive acidic pH 6 conditions.

Schaffer's group reported an iPSCs 3D culture system with pNIPAAm-PEG hydrogels based on a physical thermo-reversible gelation.¹⁹ However, physical thermo-reversible materials are usually based on loose gelation strengths, and only under high concentration, they can support iPSCs culture. While under high concentrations, thermo-reversible materials have a strong preference for a defined medium and are even not stable in pure PBS solution.²⁰

Obviously, despite all of these attempts show some attractive aspects, further improvement is still needed to provide an optimized synthetic niche.

It is an object of the present invention to provide novel hydrogels that can be used as artificial stem cell microniches and that overcome the disadvantages of prior art. In particular, it is an object of the present invention to provide hydrogels being suited as microniches having a better biocompatibility and controlled release properties allowing harvesting expanded cells under mild conditions, reducing the risk of damaging the expanded cells.

This object is achieved by a thermoresponsive hydrogel having the features of claim 1. Such a thermoresponsive hydrogel comprises dendritic polyglycerol (dPG) units and poly(N-isopropylacrylamide)-co-polyethylene glycol (pNIPAAm-co-PEG) units that are covalently bound to each other via a linker. The linker can be a cyclic triazol moiety or a diazacyclohexadiene moiety. The linker is formed by an in situ click reaction of appropriate precursor molecules.

Such a thermoresponsive hydrogel can work as 3D artificial microniche for growth and controlled release of pluripotent stem cells. This thermoresponsive hydrogel may be used for supporting pluripotent stem cells grow and expansion. It allows a controlled release of the proliferated cells out of the niches only by adjusting environment temperature under mild conditions. This hydrogel is designed and arranged to be degradable under adjustable conditions. In order to achieve this aim, a physical-chemical cogelation strategy was applied.

First, under a first temperature such as a temperature of 10 °C to 26 °C or room temperature, the dPG units and the pNIPAAm-co-PEG units are chemically connected to each other by covalent bonds. After being transferred to culture conditions (e.g., 36 °C to 38 °C, in particular 37 °C), in which the temperature was higher than the lower critical solution temperature (LCST), the pNIPAAm-co-PEG polymers then undergo physical gelation and further form a reversible hydrogel. This physically gelled hydrogel offers highly appropriate growth conditions for cells. After lowering the temperature below the LCST, the physical gelation is reversed and the cultivated cells can be easily harvested.

In an embodiment, the thermoresponsive hydrogel comprises at least one of a cyclo(-RGDfK) peptide (cyclo-(Arg-Gly-Asp-D-Phe-Lys)) and a cyclo(-RGDfk) peptide (cyclo-(Arg-Gly-Asp-D-Phe-D-Lys)) covalently bound to one of the dendritic polyglycerol units or the poly(N-isopropylacrylamide)-co-polyethylene glycol units. Both cyclic peptides act as cell binding peptides and enhance the binding ratio of cells to the hydrogel.

In an embodiment, a molar ratio between N-isopropylacrylamide (pNIPAAm) and poly(ethylene glycol) (PEG) in the poly(N-isopropylacrylamide)-co-polyethylene glycol (pNIPAAm-co-PEG) units lies in a range of from 30:1 to 1:1, in particular of from 25:1 to 2:1, in particular of from 20:1 to 3:1, in particular of from 15:1 to 4:1, in particular of from 10:1 to 5:1, in particular of from 9:1 to 6:1, in particular of from 8:1 to 7:1. Thus, it is possible that the number of pNIPAAm molecules varies between 30 times the number of PEG molecules and the same number of PEG molecules.

In an embodiment, a molar ratio between the dendritic polyglycerol units and the poly(N-isopropylacrylamide)-co-polyethylene glycol units lies in a range of from 1:3 to 1:1, in particular of from 1:2 to 1:1.5. Thus, the number of pNIPAAm units is at least as big as the number of dPG units, but can be even three times as big as the number of dPG units.

In an embodiment, the thermoresponsive hydrogel has an elastic modulus G', measured at 25 °C or at 37 °C with an 8-mm plate, in particular with an 8-mm upper plate and a 65-mm lower plate (PU08:PL65), in particular by applying a normal force of 0.05 N to 0.1 N, lying in a range of from 15 Pa to 1500 Pa. The elastic modulus can be determined, e.g., by a Kinexus Rheometer as explained in more detail in the section "(18) Rheology". In an embodiment, the elastic modulus lies in a range of from 20 Pa to 1250 Pa, in particular of from 30 Pa to 1100 Pa, in particular of from 40 Pa to 1000 Pa, in particular of from 50 Pa to 900 Pa, in particular of from 60 Pa to 800 Pa, in particular of from 70 Pa to 700 Pa, in particular of from 80 Pa to 600 Pa, in particular of from 90 Pa to 500 Pa, in particular of from 100 Pa to 400 Pa, in particular of from 150 Pa to 350 Pa, in particular of from 200 Pa to 300 Pa.

In an embodiment, the elastic modulus is higher at 37 °C than at 25 °C for the same thermoresponsive hydrogel. In an embodiment, the thermoresponsive hydrogel has a first elastic modulus G' at 25 °C lying in a first range of the above mentioned elastic modulus ranges and a second elastic modulus G' at 37 °C lying in a second range of the above mentioned elastic modulus ranges, wherein the second range covers higher values than the first range. In an embodiment, the lowest value of the second range is higher than the biggest value of the first range.

In an aspect, the present invention relates to the use of a thermoresponsive hydrogel according to the preceding explanations for cultivating cells in vitro

In an embodiment, the cells to be cultivated are induced pluripotent stem cells (iPSC).

In an aspect, the present invention relates to a method for cultivating cells in vitro

This method comprises the steps explained in the following.

First, cells to be cultivated are mixed with a thermoresponsive hydrogel according to any of the preceding explanations. Alternatively, the cells to be cultivated are mixed with a dendritic polyglycerol polymer (dPG) derivative and a poly(N-isopropylacrylamide)-co-polyethylene glycol (pNIPAAm-co-PEG) derivative that spontaneously form a thermoresponsive hydrogel according to the preceding explanations. As a result, a mixture of the thermoresponsive hydrogel and the cells to be cultivated is obtained. In this context, the mixing is carried out at a first temperature, wherein the first temperature lies below a transition temperature of the thermoresponsive hydrogel. The transition temperature of the thermoresponsive hydrogel lies in a range of from 29 °C to 35 °C, in particular of from 30 °C to 34 °C, in particular of from 30.5 °C to 33 °C, in particular of from 31 °C to 32°C.

Afterwards, the mixture of the thermoresponsive hydrogel and the cells to be cultivated is incubated at the first temperature for a first period of time.

Subsequently, the temperature of the mixture of the thermoresponsive hydrogel and the cells to be cultivated is increased to a second temperature. In this context, the second temperature lies above the transition temperature of the thermoresponsive hydrogel.

Afterwards, the cells to be cultivated are cultivated at the second temperature for a second period of time. As a result, a mixture of the thermoresponsive hydrogel and cultivated cells is obtained.

Then, the temperature of the mixture of the thermoresponsive hydrogel and the cultivated cells is decreased to a third temperature. In this context, the third temperature lies below the transition temperature of the thermoresponsive hydrogel.

Afterwards, the cultivated cells separated from the mixture of the thermoresponsive hydrogel and the cultivated cells at the third temperature.

Finally, the cultivated cells are harvested.

In an embodiment, the cells to be cultivated are induced pluripotent stem cells.

In an embodiment, a seeding concentration lying in a range of from 1×10⁴ cells/mL to 1×10⁸ cells/mL, in particular of from 1×10⁵ cells/mL to 1×10⁷ cells/mL, in particular at or around 1×10⁶ cells/mL is chosen. It could be shown that such a concentration is the most reasonable seeding density for high efficiency expansion of cells, such as iPSCs, in the niche culture system formed by the thermoresponsive hydrogel.

In an embodiment, the first period of time is a time period ranging from 1 minute to 4 weeks, in particular from 2 minutes to 3 weeks, in particular from 3 minutes to 2 weeks, in particular from 4 minutes to one week, in particular from 5 minutes to 6 days, in particular from 10 minutes to 5 days, in particular from 15 minutes to 4 days, in particular from 20 minutes to 3 days, in particular from 25 minutes to 2 days, in particular from 30 minutes to one day, in particular from 45 minutes to 20 hours, in particular from 1 hour to 15 hours, in particular from 2 hours to 12 hours, in particular from 3 hours to 10 hours, in particular from 4 hours to 9 hours, in particular from 5 hours to 8 hours, in particular from 6 hours to 7 hours. Alternatively, the first time period is a time falling within any of the precedingly mentioned time periods.

In an embodiment, the second period of time is a time period ranging from 1 day to 4 weeks, in particular from 2 days to 3 weeks, in particular from 3 days to 2 weeks, in particular from 4 days to 1 week, in particular from 5 days to 6 days. Alternatively, the second time period is a time falling within any of the precedingly mentioned time periods.

In an embodiment, the dendritic polyglycerol polymer (dPG) derivative is dendritic polyglycerol bicyclononyne (dPG-BCN). Additionally or alternatively, the poly(N-isopropylacrylamide)-co-polyethylene glycol (pNIPAAm-co-PEG) derivative is poly(N-isopropylacrylamide)-co-polyethylene glycol azide (pNIPAAm-co-PEG-N₃). Upon mixing, dPG-BCN and pNIPAAm-co-PEG-N₃ spontaneously form a hydrogel by strain-promoted azide-alkyne cycloaddition (SPAAC) reaction. This is a particularly simple and easy approach for manufacturing the Thermoresponsive hydrogel. If the cells to be cultivated are mixed with the hydrogel precursors dPG-BCN and pNIPAAm-co-PEG-N₃, the thermoresponsive hydrogel is formed in situ and embeds the cells to be cultivated in a particular appropriate way.

In an embodiment, the third temperature corresponds to the first temperature. Thus, the thermoresponsive hydrogel is after cultivation of the cells brought to the same temperature at which it was prepared before the cultivation step. This temperature can be, e.g., room temperature. Then, it is particularly easy to prepare the hydrogel and to harvest the cells afterwards under standard laboratory conditions (without any specific heating or cooling), wherein only the step of cultivating the cells is performed at a temperature (and particularly a defined carbon dioxide concentration) that is particularly appropriate for cell cultivation.

In an embodiment, the first temperature and/or the third temperature lie in a range of from 10°C to 26 °C, in particular of from 15 °C to 25 °C, in particular of from 17 °C to 23 °C, in particular of from 19 °C to 21 °C. Alternatively or additionally, the second temperature lies in a range of from 36 °C to 38 °C, in particular of from 36.5 °C to 37.5 °C, in particular at or around 37 °C.

In an embodiment, the cell cultivation is carried out with a defined carbon dioxide concentration, wherein the carbon dioxide concentration lies in a range of from 3 % to 8 %, in particular of from 4 % to 7 %, in particular of from 5 % to 6 %, in particular of from 4.5 % to 5.5 %. These and other percentages indicated in the present disclosure relate - if not explicitly stated otherwise - to volume percent (vol/vol).

In an embodiment, the thermoresponsive hydrogel in the mixture of the thermoresponsive hydrogel and the cells to be cultivated further comprises at least one of a cyclo(-RGDfK) peptide (cyclo-(Arg-Gly-Asp-D-Phe-Lys)) and a cyclo(-RGDfk) peptide (cyclo-(Arg-Gly-Asp-D-Phe-D-Lys)) covalently bound to one of the dendritic polyglycerol units or the poly(N-isopropylacrylamide)-co-polyethylene glycol units of the thermoresponsive hydrogel. These peptides serve for stimulating cell adhesion inside the hydrogel microniche. These cellular binding peptide sequences act as a biochemical signal and support cell survival and proliferation, as generally previously described.^{15b, 21}

In an embodiment, the separating of the cultivated cells is carried out by centrifuging the mixture of the thermoresponsive hydrogel and the cultivated cells. A centrifugation speed lying in a range of from 100 rpm to 1500 rpm, in particular of from 200 rpm to 1400 rpm, in particular of from 300 rpm to 1300 rpm, in particular of from 400 rpm to 1200 rpm, in particular of from 500 rpm to 1100 rpm, in particular of from 600 rpm to 1000 rpm, in particular of from 700 rpm to 900 rpm is particularly appropriate.

In an aspect, the present invention relates to a method for manufacturing a thermoresponsive hydrogel according to the preceding explanations. This method comprises the steps explained in the following.

A dendritic polyglycerol polymer (dPG) derivative and a poly(N-isopropylacrylamide)-co-polyethylene glycol (pNIPAAm-co-PEG) derivative are mixed. These polymer derivatives spontaneously form a thermoresponsive hydrogel according to the preceding explanations.

The mixing is carried out at a first temperature, wherein the first temperature lies below a transition temperature of the thermoresponsive hydrogel. In this context, the transition temperature of the thermoresponsive hydrogel lies in a range of from 29 °C to 35 °C, in particular of from 30 °C to 34 °C, in particular of from 30.5 °C to 33 °C, in particular of from 31 °C to 32°C.

Under the physical-co-chemical synergistic gelation conditions employed by the present invention, the formed dPG-pNIPAAm-co-PEG can mimic the physical property of the ECM. In an embodiment, rfRGD was introduced as biochemical signal to promote cell attachment.^{15b, 21} After all the foremost parameters about precursor ratios (e.g., dPG-BCN and pNIPAAm-co-PEG-N₃), elastic modulus (physical stiffness),^{10, 11} and cell seeding density were optimized, an optimized condition for cell culture was achieved.

Therefore, the thermoresponsive hydrogel based 3D microniche culture system could support fast expansion of cells, in particular iPSCs, and maintain high quality about proliferation capacity and high pluripotency, which can be characterized for iPSCs by the test of Ki-67 staining,²² alkaline phosphatase (ALP) staining,²³ and pluripotency immunostaining.^{1a, 19, 24}

Upon cooling down to ambient temperature (< LCST), the physical gelation effect disappeared leaving alone the covalent bond based gelation effect. This mechanism precisely benefits the culture system with the ability of controlled release of cells, making it possible to harvest them with high yield. Overall, aspects of the present invention provide an advanced controlled release hydrogel scaffold with great utility for 3D cell culture, in particular 3D iPSCs culture.

Native ECM, which works as a key constitutive part of the microniche and plays an essential role in regulating cell behavior, is a rather complex system.¹¹ Establishing an artificial niche microenvironment by mimicking the physical and biochemical characteristics of such an extracellular matrix involves the optimization of several parameters, for example, chemically defined cell-compatible material synthesis, hydrogel stiffness, cell seeding density, and adhesion-relative biochemical cues.²⁵

The design and the specific preparation of the synthetic precursors are the primary steps for manufacturing artificial ECM-mimicking environments.²⁶ Polyethylene glycol (PEG)^{14c, 27} and dendritic polyglycerol (dPG)¹⁸ were chosen here because of their high biocompatibility, low batch-to-batch variability, as well as their readily amenable scalability. The have also previously shown great promise to act as cellular scaffolds.²⁸

A specific example for manufacturing a thermoresponsive hydrogel according to an embodiment of the present invention comprises the following steps:
a) The biocompatible poly(N- isopropylacrylamide)-co-polyethylene glycol polymers were synthesized by chain reaction polymerization with NIPAAM and poly(ethylene glycol)-acrylate under the initiator molecule of AlBN. After purification, the achieved poly(NIPAAm-co-PEG) was mesylated with MsCI and azidated with NaN₃, and finally formed poly(NIPAAm-co-PEG)-N₃.
b) The dPG-(BCN)_{6%} was synthesized in four steps starting with dPG (Mn = 6 kDa): mesylation, azidation, reduction of azides with PPh3 and then coupling with the BCN derivative. Azide functionalized dPG was synthesized in two steps, mesylation and substitution by azide following the procedure as reported before.³¹ BCN-RGD was synthesized based on the modification of BCN with cyclic pentapeptide c[RGDfk], which was linked to BCN through its lysine residue without hindering its biological performance.³²
c) Two sets of aqueous precursor solutions with different concentrations were prepared by dissolving ultra-pure precursors of pNIPAAm-co-PEG-N₃ obtained in step a) and dPG-BCN obtained in step b) (with BCN-RGD, and the final concentration of c[RGDfk] in precursors mixtures was kept as 1mM ^{32a}) in cell medium separately.
d) Together with (or without) iPS cells (which were trypsinized with accutase solution into single cells) in culture medium with varying densities, the pNIPAAmco-PEG azide and dPG-BCN (with BCN-RGD) precursor solutions obtained in step c) were mixed in EP tubes under series of different parameters (concentrations and ratios).
e) Then, the niche with cells seeded inside (obtained in step d)) were immediately transferred from the EP pipe to the culture dish plates. After that, the culture dish plates were kept at room temperature for 20 minutes (during this period, chemical gelation happens by mechanism of SPAAC reaction).
f) After addition of cell medium, the plates with niche gels were then transferred into a carbon-dioxide cell incubator at 37 °C (physical gelation happens).
g) Finally, a chemical and physical co-gelation system was formed inside the niche and cells were just seeded at the same time in the microniches and then cultured in the cell incubator (37° C).
h) After being cultured for a certain time (at 37 °C with 5% CO₂), the cell expansion niche system could be transferred to room temperature (25 °C) conditions again for 5 min. During this time the physical gelation disappeared and thus only chemical gelation left in the niche system, so the niche was loosened, and expanded cells could be released out of the niches. After centrifuging (400r/min) for 5 mins, the pure cells were harvested.
i) Cell quality characterization was performed with Ki-67 staining test and the immunocytochemistry analysis of pluripotency markers to investigate their proliferation ability and whether the harvested cells maintained their pluripotency.

According to aspects of the present invention, stimuli-responsive polymer materials were used as powerful tools in establishing a dynamic or controlled reversible microenvironment, especially in cell biology. Among various stimuli triggers, thermoresponsiveness can be easily controlled and is the most promising one to engineer materials for cell loading and release. Herein, poly(N-isopropylacrylamide) (PNIPAM), which has been widely applied as a temperature-sensitive polymer with reversible gelation at the lower critical solution temperature (LCST) of 32 °C^{14b, 29} was chosen as one of the constituents of the thermoresponsive hydrogel. In order to combine the advantages of both, a pNIPAAm-co-PEG polymer was synthesized here as one polymer for making up the thermoresponsive hydrogel.

According to an aspect of the present invention, for the chemical gelation forming, bicyclooctyne and azide functional group were functionalized onto the end of branches of the dPG and the end of the pNIPAAm-co-PEG polymers separately and could be used in the *in situ* bioorthogonal SPAAC reaction.

According to an aspect of the present invention, benefiting from the cogelation strategy, dPG-pNIPAAm-PEG hydrogel networks serve as the backbone of the microniche for supporting cells better than the pNIPAAm-co-PEG hydrogel networks. On one hand, they overcome the disadvantage caused by a single pNIPAAm-co-PEG polymer (i.e., a purely physically crosslinked hydrogel), namely, failing to show strength and being not stable enough to serve as a cell scaffold. On the other hand, they still make use of pNIPAAm, which has been equipped with microniche controlled-release properties.

According to aspects of the present invention, stiffness characterization was performed on the hydrogel microniches formed with various precursors' ratios and concentrations, as substrate stiffness, typically characterized by the elastic modulus, is one of the important mechanical features in controlling cell fate. Because the hydrogels-based niche was constructed in an embodiment by two precursor polymers, i.e. pNIPAAm-co-PEG-N₃ and dPG-BCN, the mechanical stiffness of the hydrogel networks could be regulated by the respective ratios and overall concentration of these two precursors.

According to aspects of the present invention, the performance of iPSCs' response to the precursors' ratios of materials (in the thermoresponsive hydrogel) for establishing a 3D niche environment was tested to invest the performance of the thermoresponsive gels as microniches for cell growth and get the relative optimized parameters. The artificial extracellular niche's interior morphology analysis was investigated firstly, and the cell survival, morphology features and proliferation were also monitored by live/dead staining.

According to aspects of the present invention, as the mechanical feedback of the linkage between cell and substrate play a key role in regulating stem-cell fate, iPSCs' viability in dependence on the stiffness and precursor content of the 3D gel niche environments was tested. Thus, it was investigated how the cells are influenced by the topography and stiffness in the artificial niche environment. Since polymer concentration or crosslinking density influences topography and hydrogel stiffness, morphological properties of the niche environment with various polymers were adapted to test how their morphology was affected by the crosslinking density.

According to aspects of the present invention, to circumvent the stiffness change caused by ratio variation, all the niche environment was performed by SEM. In order to assess whether the different stiffness of the niche scaffold affected iPSCs' survival and proliferation, iPSCs were embedded into hydrogels precursors with high, medium, low, and really low concentrations, respectively, of which the elastic moduli ranged from 1070 to 17.8 Pa accordingly. Amount these, the optimized elastic modulus could be chosen as the standard stiffness parameters for the dPG-pNIPAAm-co-PEG-based 3D niche environment culture system.

In the extracellular environment, cells do not live alone. They can secrete substances that affect surrounding cells and keep sensing with each other.^{11, 30} Thus, cell distribution and seeding density work as crucial determiners that not only affect the cell-cell contact but also affect cells' survival and expansion in the artificial niche environment.

According to aspects of the present invention, overall performance of the thermoresponsive hydrogel (dPG-pNIPAAm-co-PEG) 3D niche culture system in supporting iPSCs' proliferation was assessed by investigating the cell viability and expansion efficiency.

According to aspects of the present invention, to further investigate the relationship between the iPSCs' seeding density and expansion efficiency in this prepared niche environment and finally determine the optimum seeding density, cells with different (high, medium, low) concentrations were separately seeded in three scaffold niches with the same elastic modulus and specific precursor ratios. Furthermore, the morphology of the cells that survived in the artificial niche environment was monitored. Overall, considering all the possible factors, an optimized seeding concentration could be chosen as the most reasonable seeding density for the iPSCs expansion in the presently described niche culture system.

During the controlled release and cell harvest process, to release the expanded cells, the culture medium was replaced by phosphate-buffered saline (PBS), and the culture dish was incubated at 25 °C (< LCST) for 5 min. Then, due to the reversibility of the physical gelation process, the thermo-gelation disappeared, leaving behind a loose network of covalently crosslinked gel. After the morphology of the niche scaffold changed from white turbid solid to transparent and colorless, cells and niche scaffold were together transferred into a centrifuge tube. By centrifugation, iPSCs' embryonal bodies (EB) that were formed in the niche environment were separated, collected, and thus finally harvested.

All embodiments of the thermoresponsive hydrogel can be combined in any desired manner and can be transferred either individually or in any arbitrary combination to the described use and the described methods. Likewise, all embodiments of the described use can be combined in any desired manner and can be transferred either individually or in any arbitrary combination to the thermoresponsive hydrogel and to the described methods. Furthermore, the embodiments of the described methods can be combined in any desired manner and can be transferred either individually or in any arbitrary combination to the thermoresponsive hydrogel, to the use, and to the respective other method.

Further aspects and details of the invention will be explained with respect to Figures and exemplary embodiments. In the Figures:

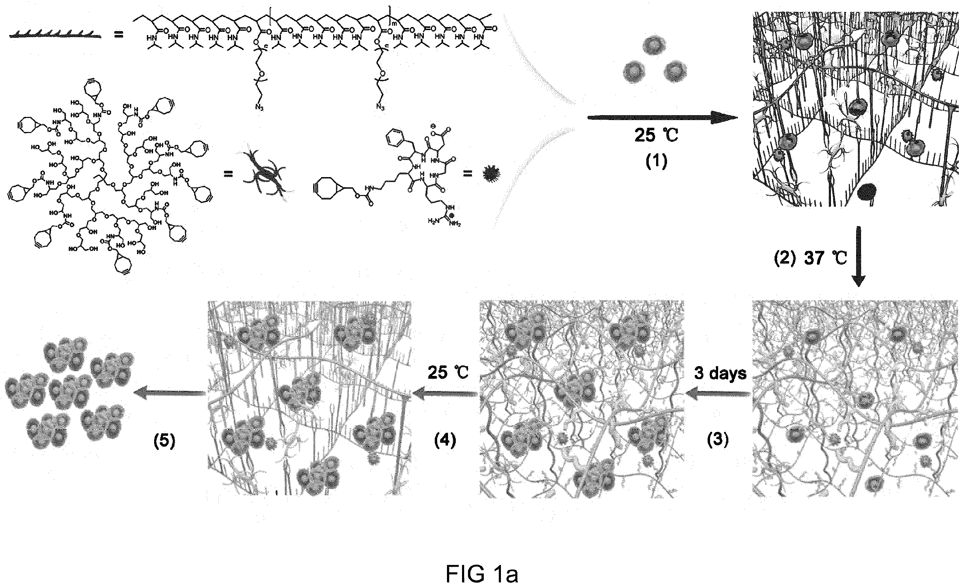

- FIG 1a: shows a schematic representation of the mechanism of a 3D culture of iPSCs in a thermoresponsive hydrogel;

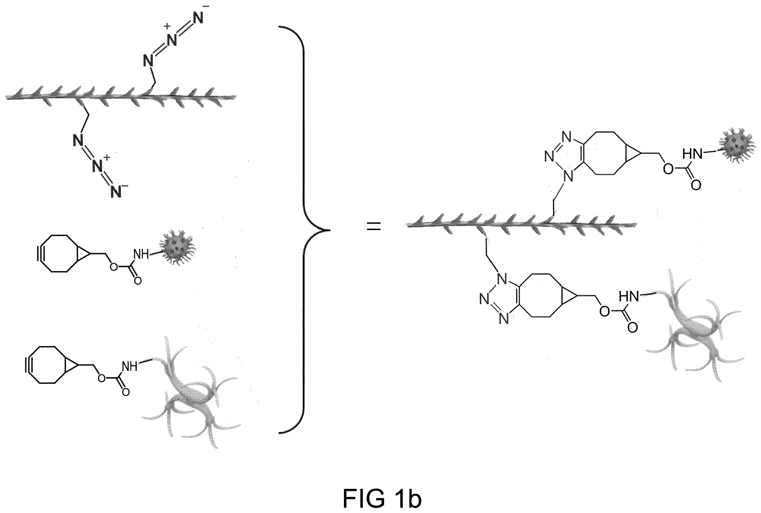

- FIG 1b: shows a reaction scheme of an in situ formation of a cyclic triazol linker;

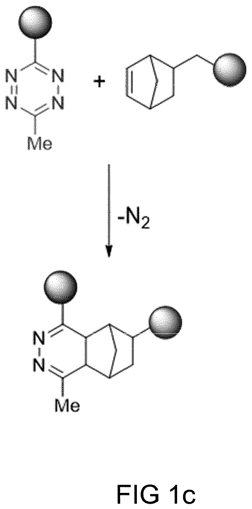

- FIG 1c: shows a reaction scheme of an in situ formation of a diazacyclohexadiene linker;
- FIG S1: shows a ¹H NMR (500 MHz, D₂O) spectrum of poly(NIPAAm-co-PEG);
- FIG S2: shows a ¹H NMR (500 MHz, D₂O) spectrum of poly(NIPAAm-co-PEG)-OMs (mesylated poly(NIPAAm-co-PEG));
- FIG S3: shows a ¹H NMR (500 MHz, D₂O) spectrum of poly(NIPAAm-*co*-PEG)-N₃ at different temperatures;
- FIG S4: shows an infrared spectrum of poly(NIPAAm-co-PEG)-N₃ (v = 2097 cm⁻¹ (N₃));
- FIG S5: shows a GPC analysis for poly(NIPAAm)-co-PEG polymers;
- FIG S6: shows a GPC analysis for dPG;
- FIG S7: shows a ¹H NMR spectrum of dPG-OMs;
- FIG S8: shows a 1H NMR spectrum of dPG-N₃;
- FIG S9: shows a ¹H NMR spectrum of dPG-NH₂;
- FIG S10: shows infrared spectra of dPG-OMs, dPG-N₃, dPG-NH₂;
- FIG S11: shows a ¹H NMR spectrum of dPG-BCN;
- FIG 2: shows the synthesis of precursors of an embodiment of the thermoresponsive hydrogel and a fabrication process of the embodiment of the thermoresponsive hydrogel;
- FIG 3a: shows the elastic modulus G' and viscous modulus G" for various hydrogels with variable volume ratio of dPG-BCN to pNIPAAm-co-PEG-N₃ (*c*_{pNIPAAm-co-PEG-N3} = 100 mg mL⁻¹, *c*_{dPG-BCN} = 100 mg mL⁻¹) at a constant temperature (37 °C);
- FIG 3b: shows the time-dependent gelation test for system with four different precursor concentrations under a given ratio (1:1);
- FIG 3c: shows the rheology data of hydrogels under various precursor ratios;
- FIG 3d: shows the rheology data of hydrogels (gel 1 to gel 4) with various precursor concentration ratios;
- FIG 3e: shows a first plot of the rheology contribution from different gelation mechanisms of thermoresponsive hydrogels at different temperatures;
- FIG 3f: shows a second plot of the rheology contribution from different gelation mechanisms of thermoresponsive hydrogels at different temperatures;
- FIG 4a: shows SEM morphology in response to different precursor ratios of the hydrogel, wherein the scale bar indicates 40 µm;
- FIG 4b: shows the cell viability tests depending on the precursor ratios of the hydrogel monitored by live/dead staining, wherein the scale bar indicates 100 µm;
- FIG 4c: shows the iPSCs proliferation as performance response to different precursor ratios of the hydrogel;
- FIG 5a: shows the SEM morphology in response to the stiffness and precursor content of the hydrogels, wherein the scale bar indicates 40 µm;
- FIG 5b: shows the cell viability in response to the stiffness and precursor content of the hydrogels monitored by bright field microscopy, wherein the scale bar indicates 100 µm;
- FIG 5c: shows the EB size distribution and frequency on different culture days;
- FIG 5d: shows the iPSCs proliferation efficiency in response to the stiffness of the hydrogel;
- FIG 6a: shows the cell viability test in dependence on the cell seeding density monitored by bright field microscope (scale bar = 100 µm);
- FIG 6b: shows the EB size distribution and frequency for different cell seeding densities on different culture days;
- FIG 6c: shows the iPSCs proliferation efficiency response to culture time under different cell seeding densities in the hydrogel;
- FIG 7a-c: show the cell viability and morphology during the culture process at day 1, day 3, and day 4, respectively;
- FIG 7d-e: shows the cell viability test at day 1 and day 3 monitored by live/dead staining;
- FIG 7f: shows the iPSCs proliferation efficiency under optimized conditions;
- FIG 8a: shows the iPSCs mixed with precursors and embedded into the culture system and cultured for a cultivation time;
- FIG 8b: shows a step in which the culture medium was replaced by PBS, and the culture dish was incubated at 25 °C (< LCST) for 5 min to release the expanded cells;
- FIG 8c: shows the iPSCs EB purification and collection by centrifugation;
- FIG 8d: shows the EB cells were cultured for three days (scale bar = 100 µm);
- FIG 8e: shows the Alkaline phosphatase (ALP) staining (scale bar = 50 µm);
- FIG 9: shows the Ki-67 staining of the iPSCs EB after having been cultured for 1, 3, and 5 days, respectively (scale bar = 100 µm);
- FIG 10: shows the immunostaining of the spheroids indicating that the cells expressed the pluripotent markers of Nanog, Sox2, Oct4, and SSEA1; and
- FIG 11: immunostaining confirming in vitro differentiation of iPSCs into all three germ layers.

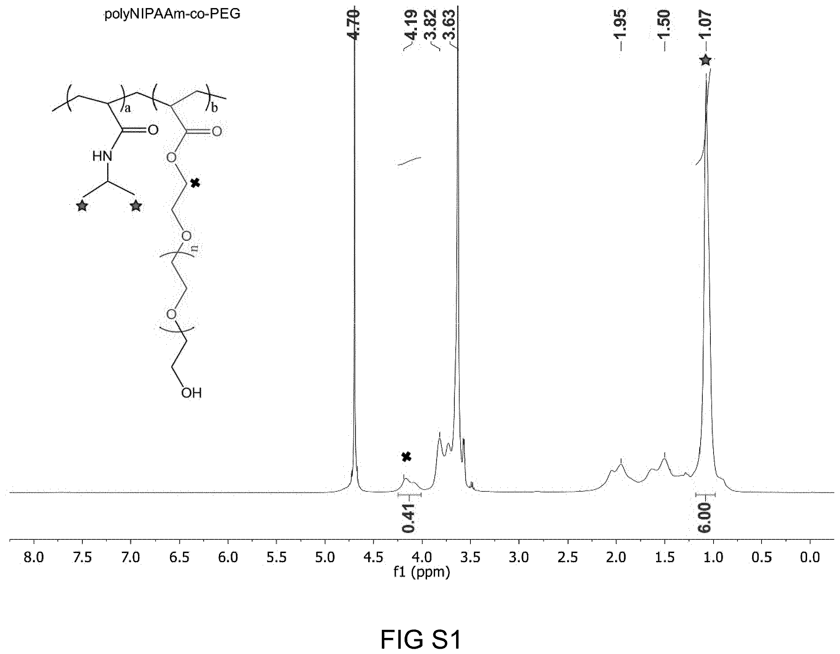

A synthetic and controlled release iPSCs' 3D artificial niche environment according to a physical-chemical cogelation strategy for iPSCs culture was manufactured according to a working mechanism schematically represented in Figure 1.

A 3D microenvironment was tailored with two specially designed biocompatible linkers (polymers) in two steps: dPG-bicyclononyne (dPG-BCN) and poly(N-isopropylacrylamide) co-polyethylene glycol azide (pNIPAAm-co-PEG-N₃). Additionally, rfRGD was introduced as biochemical signal to promote cell attachment.

Firstly, step (1) of Figure 1 shows that, under room temperature, the two polymers can form a hydrogel by strain-promoted azide-alkyne cycloaddition (SPAAC) reaction. Only chemical gelation (due to covalent bonds between the polymers) was present. After being transferred to culture conditions (37 °C; step (2)), in which the temperature was higher than the lower critical solution temperature (LCST), the N-isopropylacrylamide co-polyethylene glycol polymers undergo physical gelation and further form a reversible hydrogel. Under physical-co-chemical synergistic gelation conditions, the formed dPG-pNIPAAm-co-PEG can mimic the physical property of the ECM.

All the foremost parameters about precursor ratios, elastic modulus (physical stiffness), and cell seeding density were optimized, achieving a highly appropriate condition for cell culture. The culture system could support iPSCs' fast expansion and maintain high quality of proliferation capacity and high pluripotency, which can be further characterized. The hydrogel formed a stable niche by physical-chemical cogelation strategy, which supported iPSCs' survival and quick expansion.

After being cultured for 3 days (step (3)), the system was transferred to 25 °C again (step (4)). Due to this temperature decrease, the physical gelation disappeared, the niche was loosened, and then the cells were released from the niches.

The final cell harvest process was done by centrifugation (step (5)).

Figure 1b shows a reaction scheme of an in situ formation of a cyclic triazol linker. The starting materials are dPG-bicyclononyne (dPG-BCN) and poly(N-isopropylacrylamide) co-polyethylene glycol azide (pNIPAAm-co-PEG-N₃). For better illustration, the azide group and the bicyclononyne group are explicitly visualized in Figure 1b. Due to a click reaction of these groups, a cyclic triazol linker is formed and a covalent bond is generated between the dPG units and the pNIPAAm-co-PEG units.

FIG 1c shows a reaction scheme of an in situ formation of a diazacyclohexadiene linker that is formed in situ by a reverse Diels-Alder reaction under release of N₂. The balls represent dPG and pNIPAAm-co-PEG, respectively. "Me" denotes a methyl residue. The in situ formation of the diazacyclohexadiene linker establishes a covalent bond between the dPG units and the pNIPAAm-co-PEG units.

Both linkers serve for a good biocompatibility and biodegradability of the formed polymers.

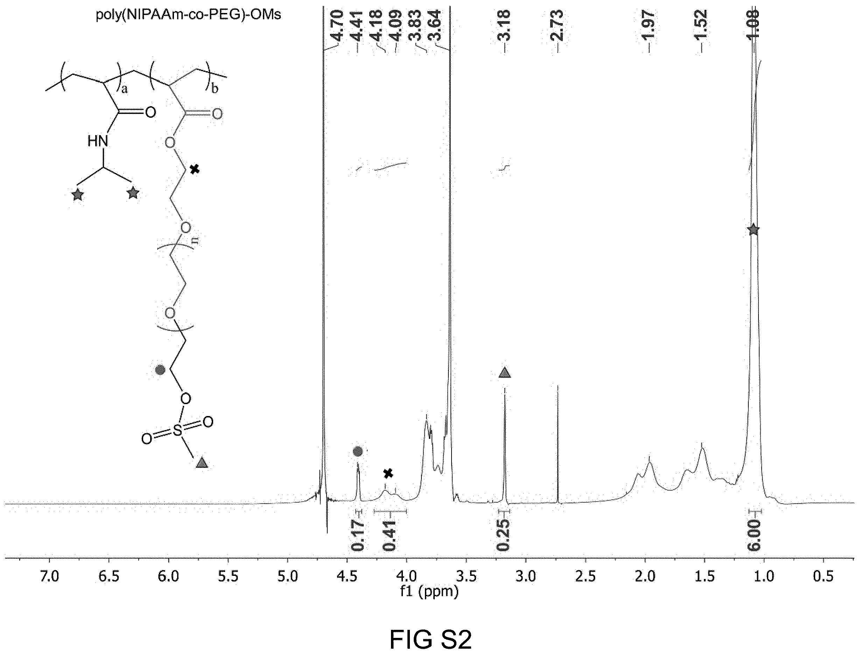

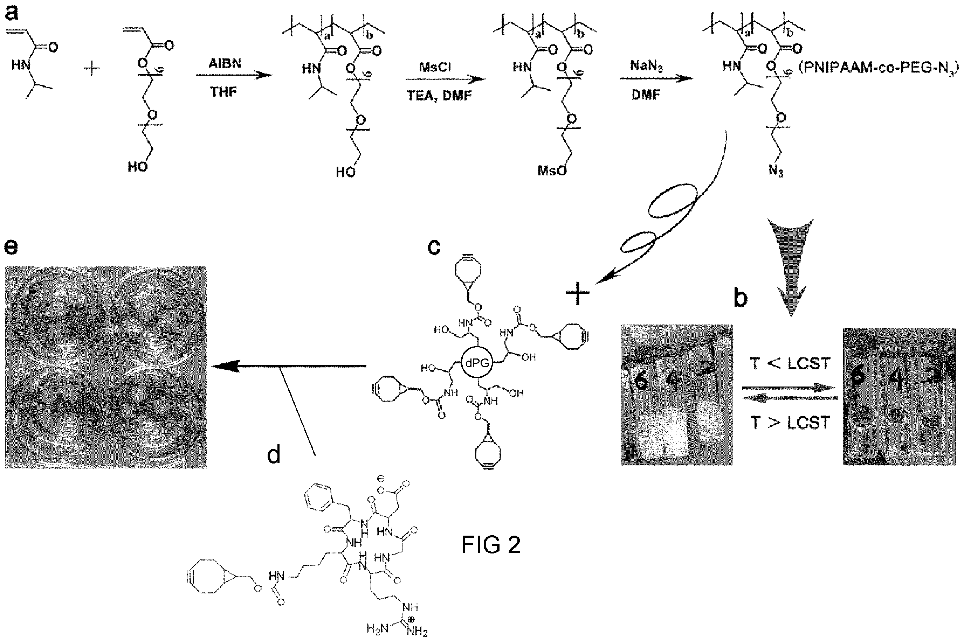

Figures S1 to S11, Figures 2 to 10, and Tables 1 to 3 will now be explained in more detail referring to exemplary embodiments.

### (1) Synthesis and characterization of dPG-bicyclononyne (dPG-BCN) and poly(N-isopropylacrylamide) co-polyethylene glycol azide (pNIPAAm-co-PEG-N₃) polymers:

*Synthesis of poly(NIPAAm-co-PEG):* The biocompatible poly(N-isopropylacrylamide)-co-polyethylene glycol polymers were synthesized by chain reaction polymerization with NIPAAM and poly(ethylene glycol) - acrylate under the initiator molecule ofAIBN. The synthesis process for this linker was according to the free-radical polymerization³³ with some optimization. To a flask filled with Argon, NIPAAM (7.49g, 66 mmol) and PEG-acrylate (4.75g, 13.2 mmol) were dissolved in 10 mL THF. Then, the flask was filled with Argon again and 394mg AIBN (dissolved in 5 mL THF) was injected by pumping under fierce stirring. The reaction was carried out at 65°C, in dark and under stirring. After 24h, the reaction mixture was precipitated directly in ice-cold Et₂O and colorless product was obtained by suction filtration. The copolymer was then dialyzed for 3 days, frozen, and lyophilized as a white solid (9.1g, yield 72%). Formation and the purity of the poly(NIPAAm-co-PEG polymer was confirmed by ¹H NMR, and the element analysis test. For the desired above product, NMR test (¹H NMR (500 MHz, D₂O) δ (ppm): Figure S1, and the element analysis test (Table 1), were done for characterization, showing successful purification. The mole ratio of NIPAAm and PEG (4.88:1 ≈ 5:1) was calculated from the integration ratio between the methyl protons (6 H for (*CH₃*)*₂*CHNHCO-) of NIPAAm and the methylene protons (2H for HOCH₂CH₂(OCH₂CH₂)n OCH₂*CH₂*OCO-) of PEG appearing at 1.07 and 4.19 ppm (Figure S1), respectively.

**Table 1 C - H - N -Element Analysis of poly(NIPAAm-co-PEG)**

| Sample no. | Element | Content | Peak area | |
|---|---|---|---|---|
| 1 (1.6140mg) | N: | 9.142 | 6237 | 0.8165 |
| | C: | 60.43 | 24682 | 1.0159 |
| | H: | 9.317 | 12415 | 0.9059 |
| 2 (1.9450mg) | N: | 9.141 | 7547 | 0.8165 |
| | C: | 60.30 | 29676 | 1.0159 |
| | H: | 9.318 | 15017 | 0.9059 |

| | | | | |
|---|---|---|---|---|
| The sample (poly(NIPAAm-*co*-PEG)) prepared for test (with a NIPAAm-PEG ratio of 5:1). | | | | |

*Synthesis of poly(NIPAAm-co-PEG)-N₃:* poly(NIPAAm-co-PEG) (5g, 5.38 mmol -OH) was firstly dried and then solved into DMF (100mL) in a flask under fierce stirring. After that, Et₃N (1.122 mL, 8.0725mmol) was added into the solution and the flask was cooled down to 0 °C with an ice bath. MsCI (0.92g, 8.07mmol) dissolved in DMF (15 mL) was added dropwise to the suspension. The reaction was performed at room temperature overnight. The color of the mixture solution changed to yellow. The product was purified by dialysis (MWCO 1 kDa) against a mixture of water and methanol. After lyophilization, the product (poly(NIPAAm-co-PEG)-OMs) was obtained after lyophilization as a white solid. The presence of mesyl groups was confirmed by ¹H (Figure S2). NaN₃ (3.50g, 53.82mmol) was added into the solution of mesylated polymer in DMF. At 65 °C, the reaction was kept stirring for 2 days. DMF was evaporated and the obtained residues was purified by dialysis using 1 kDa MWCO membrane first in water-methanol mixture and then in water for one day. Azide functionalized polymer was obtained after lyophilization of the obtained aqueous solution as a white solid (3.74g, yield 74.77 %). ¹H NMR (500 MHz,) δ (ppm) was done at different temperatures (298K -313K) and as shown in Figure S3. According to the ¹H NMR tests at different temperature, the signals of the copolymer disappeared when the temperature was up to the copolymer's LCST. Therefore, from these tests at different temperatures, it can be inferred that the LCST of the synthetic poly(NIPAAm-co-PEG)-N₃ was about 304K (31°C). Additionally, IR (Figure S4), GPC (Figure S5) and elemental analysis (Table 2) confirmed the formation and purity of the poly(NIPAAm-co-PEG)-N₃ polymer.

**Table 2 C - H - N -Element Analysis of poly(NIPAAm-co-PEG)-N₃**

| Sample no. | Element | Content | Peak area |
|---|---|---|---|
| 1 (2.0270mg) | N: | 10.07 | 5719 |
| | C: | 53.05 | 20484 |
| | H: | 8.934 | 8552 |
| 2 (1.4760mg) | N: | 10.01 | 4139 |
| | C: | 53.07 | 14979 |
| | H: | 8.927 | 5926 |

| | | | |
|---|---|---|---|
| The sample (poly(NIPAAm-co-PEG)-N₃) prepared for test (with a theoretical NIPAAm -PEG ratio of 5:1). | | | |

*Synthesis of dPG-BCN:* The dPG-(BCN)_{6%} was synthesized in four steps starting with dPG (Mn = ca. 6 kDa; Figure S6 indicating Mn: 5460 g/mol, Mw:8550 g/mol and D: 1.56): mesylation, azidation, reduction of azides with PPh3 and then coupling with the BCN derivative. Azide functionalized dPG was synthesized in two steps, mesylation and substitution by azide following the procedure as reported before³¹ (Figure S7, S8). Amine functionalized dPG was synthesized by reducing the dPG(N₃)_{6%} using PPh3 in THF-H₂O following the procedure as reported before ³⁴ (Figure S9). Conversion of azide groups to amine was also confirmed by IR by disappearance of peaks at 2100 cm⁻¹ as shown in the Figure S10. Finally, the dPG-NH₂ was reacted with Bicyclo[6.1.0[non-4-yn-9ylmethyl (4-nitrophenyl) carbonate (BCN-PNP) dPG-BCN was synthesized Figure S11.

*Synthesis of BCN-RGD:* BCN-RGD was synthesized based on the modification of BCN with cyclic pentapeptide c[RGDfK], which was linked to BCN through its lysine residue without hindering its biological performance.^{31, 32b} Cyclic Arg(Pmc)-GlyAsp(OtBu)-D-Phe-Lys(Boc) (60 mg, 0.0993 mmol)was dissolved in 24 mL DMF, followed by addition of TEA (48 µL, 3 eq). A solution of Bicyclo[6.1.0[non-4-yn-9ylmethyl (4-nitrophenyl) carbonate (32.85 mg, 0.1143 mmol, 1.05 eq) dissolved in 24 mL DMF was added into the RGD solutions and stirred for 16 hours at RT. After that, the DMF was removed by rotary evaporator, and the residue was purified by column with ethyl acetate as eluent. After that, 74.254 mg (80% yield) product was obtained.

### (2) Fabrication of the thermoresponsive hydrogel scaffold niche

*Artificial niche Fabrication:* Firstly, two sets of aqueous precursor solutions with different concentrations (Table 3) were prepared by dissolving ultra-pure precursors of pNIPAAm-co-PEG-N₃ as illustrated in Figure 2a (showing the synthesis reaction of pNIPAAm-co-PEG-N₃ with a NiPAAM : PEG-acrylate ratio of 5 : 1); and dPG-BCN (with BCN-RGD as shown in Figure 2d).

**Table 3 Crosslinker concentration related precursor concentrations for gel1- gel4.**

| Sample no. | *c*_{dPG-BCN} | *c*_{pNIPAAm-co-PEG-N3} |
|---|---|---|
| Gel 1 | 200mg mL⁻¹, | 200mg mL⁻¹, |
| Gel 2 | 100mg mL⁻¹, | 100mg mL⁻¹, |
| Gel 3 | 75mg mL⁻¹, | 75mg mL⁻¹, |
| Gel 4 | 50mg mL⁻¹, | 50mg mL⁻¹, |

FIG 2b shows the thermo-reversible phase-change phenomenon of the pNIPAAm-co-PEG-N₃ (LCST≈31 °C).

FIG 2d shows the chemical structure of RGD-BCN. The final concentration of c[RGDfK] in precursor mixtures was kept at 1mM ^{32a}) in cell medium separately. Figure 2c shows that, together with (or without) iPS cells in culture medium with varying densities, the pNIPAAmco-PEG azide and dPG-BCN (with BCN-RGD) precursor solutions were mixed in EP tubes under series of different parameters (concentrations and ratios). Then, the niche with cells seeded inside were immediately transferred from the EP pipe to the culture dish plates (5µL per drop). After that, the culture dish plates were kept at room temperature for 20 mins (chemical gelation happens by mechanism of SPAAC reaction). After addition of cell medium, the plates with niche gels were then transferred into carbon-dioxide cell incubator at 37 °C (physical gelation happens). Figure 2e shows that, finally, a chemical and physical co-gelation system was formed inside the niche (within ESGRO medium).

### (3) Stiffness characterization of the hydrogel microniche formed with various precursor ratios and concentrations

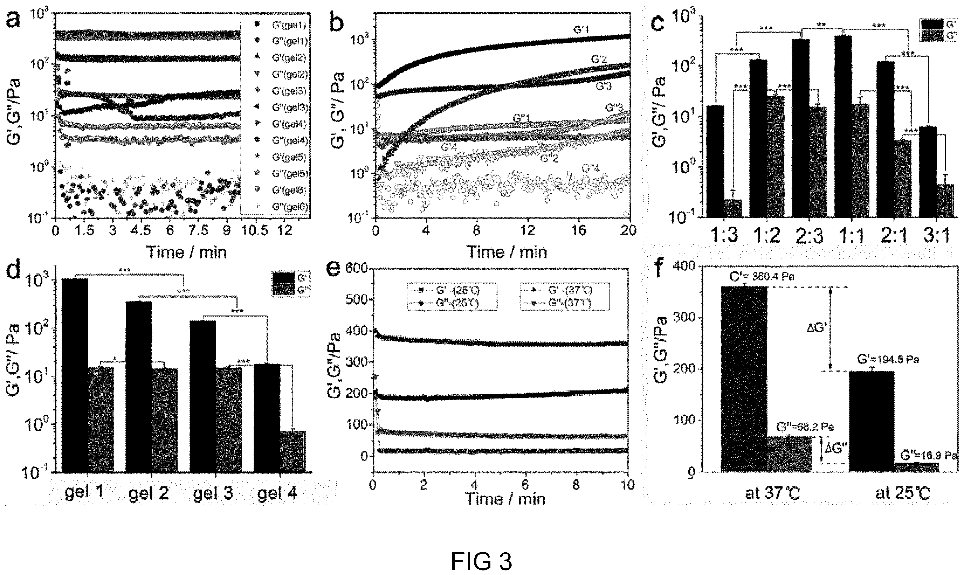

Substrate stiffness, typically characterized by the elastic modulus, is one of the important mechanical features in controlling cell fate. To tailor a microenvironment with proper physical strength, it is essential to optimize the stiffness parameters by adjusting the related factors. To assess the effect of precursor ratios, the elastic and viscous moduli were investigated by performing rheological measurements. The ratios of precursors (dPG-BCN to pNIPAAm-co-PEG-N₃) for gel 1 to gel 6 were accordingly preset as 1:3, 1 :2, 2:3, 1:1, 2:1 and 3:1, respectively as shown in Figure 3a, 3c. It could be referred from the data in Figure 3a, 3c that, only under certain precursor ratios, the synthesized hydrogel-based microniche environment could be formed with high efficiency and possess an optimized stiffness, especially when the ratio (dPG-BCN and pNIPAAm-co-PEG-N3) was 1:1 or 2:3.

Besides the precursor ratios, polymer concentration was another crucial parameter that could affect the stiffness. To further investigate the stiffness parameter affected by polymer concentration (Table 3), a rheological test was performed by varying the polymer concentrations under constant ratio (Figure 3b, 3d). The rheology data of hydrogels niche (gel 1- gel 4) and results in Figure 3b, 3d indicated that the strength of the elastic modulus was in accordance with the concentration of the polymer under a specific polymer precursor ratio. Thus, from the time-dependent elastic modulus G' and viscous modulus G" test, the effect and relationship of temperature and gelation time were also investigated during the gelation process. As shown in Figure 3b, gelation time was respectively about 20 min under the temperature of 37 °C. It demonstrated that, in consideration of the operation and 3D scaffold niche manufacture process, 20 min is the best choice for the parameters of gelation time for this application.

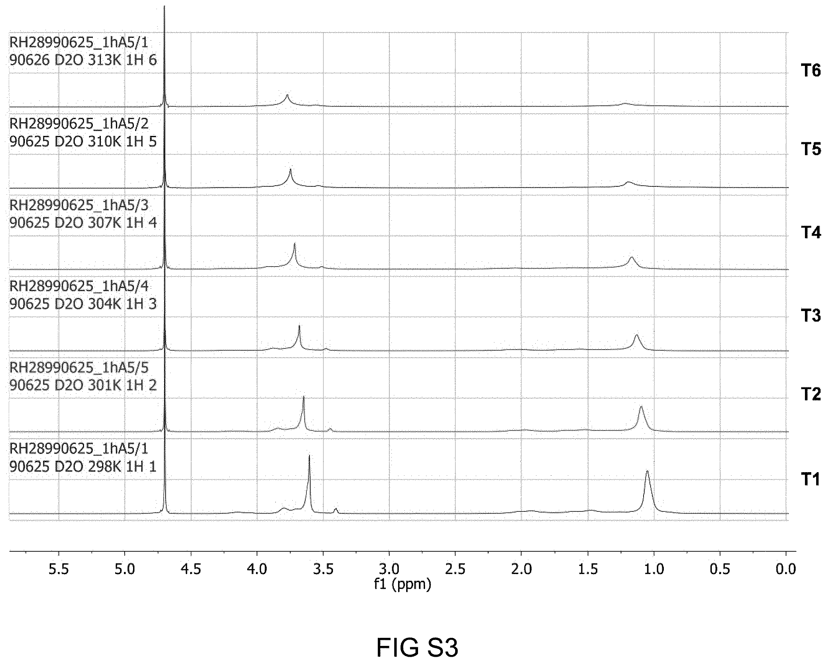

Figures 3e and 3 f show the rheology contribution from different gelation mechanisms of thermoresponsive hydrogels at different temperatures. In Figures 3a to 3f, * denotes *p* < 0.05, ** denotes *p* < 0.01, and *** denotes *p* < 0.001.

### (4) iPSCs' response to the precursors ratios of materials for establishing a 3D niche environment

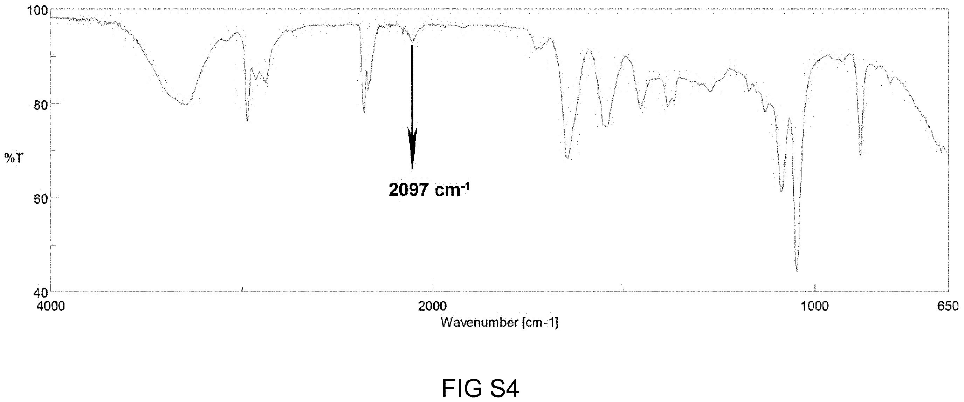

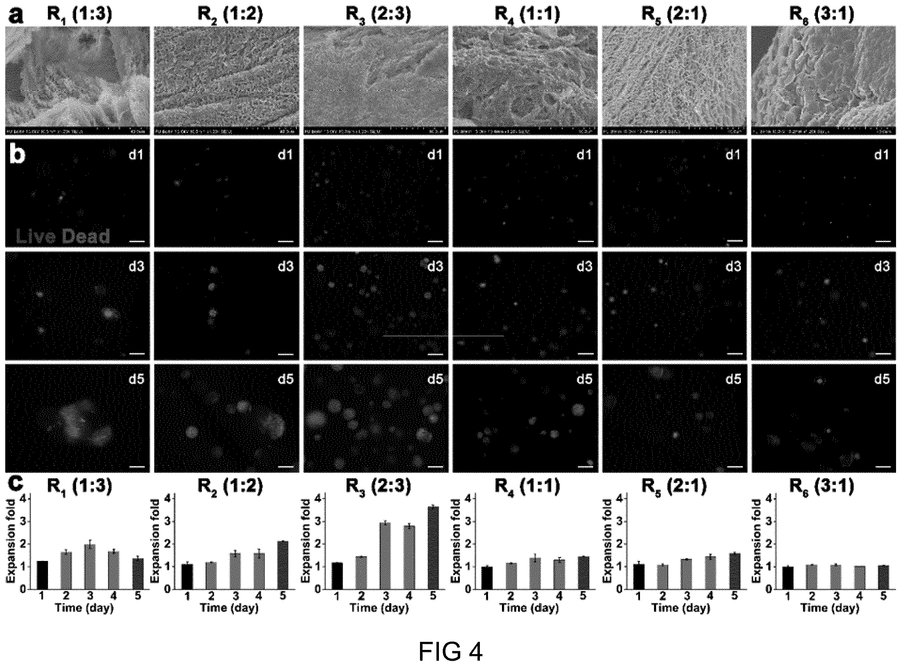

Figure 4 shows the response of iPSC viability and scaffold environment morphology to precursor ratios of the hydrogels niche.

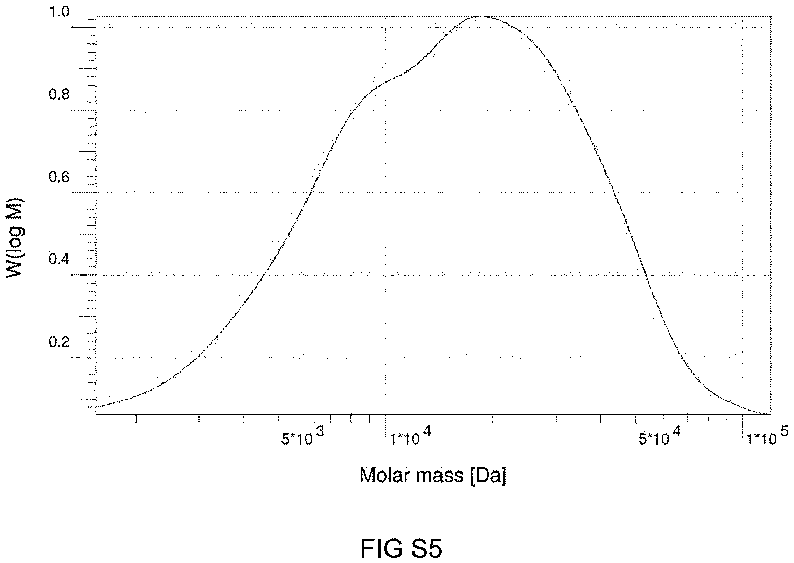

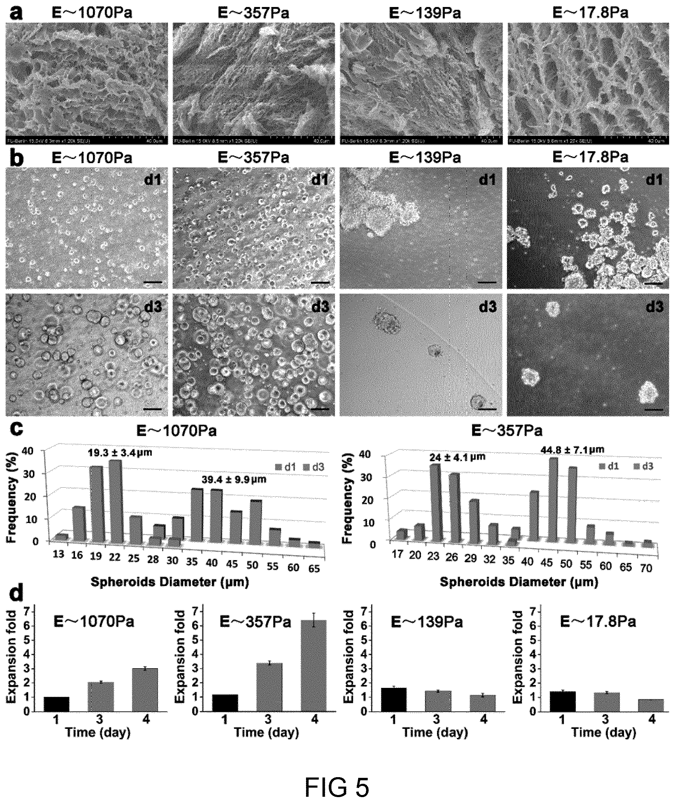

The extracellular environment varies not only in composition but also in physical parameters, including stiffness, which typically is characterized by the elastic modulus and topography. Therefore, it is essential to investigate how the cells are influenced by the topography and stiffness in the artificial niche environment. Therefore, morphological properties of the niche environment with various polymers were adapted to test how their morphology was affected by the crosslinking density. Figure 5 shows that alterations in the concentration ratio of various polymers could influence the cell fate. To be more precise, Figure 5 shows the iPSC viability and artificial niche environment's morphology in response to the stiffness and precursor content of the hydrogels. The morphology analysis (Figure 5a) of concentrations shows that the size of the hole is closely related with the gel stiffness.

In order to assess whether the different stiffness of the niche scaffold affected iPSCs' survival and proliferation, iPSCs were embedded into hydrogels precursors with high, medium, low, and really low concentrations, respectively, of which the elastic moduli ranged from 1070 to 17.8 Pa accordingly. Meanwhile, cells were cultured in the ESGRO medium, and their survival conditions were monitored at least for 3 days (Figure 5b). The results revealed that cell behavior in this artificial niche was significantly affected by the stiffness of their microenvironment. Both the size and the number of the EB shown in Figure 5c and 5d revealed that only a certain stiffness environment allows for iPSCs' survival and expansion well. Overall, this study revealed that the elastic modulus of 357 Pa could be chosen as the standard stiffness parameters for the dPG-pNIPAAm-co-PEG-based 3D niche environment culture system.

### (5) iPSCs' viability and expansion efficiency with regard to cell seeding density.

In the extracellular environment, cells do not live alone. They can secrete substances that affect surrounding cells and keep sensing with each other. Thus, cell distribution and seeding density work as crucial determiners that not only affect the cell-cell contact but also affect cells' survival and expansion in the artificial niche environment. To further investigate the relationship between the iPSCs' seeding density and expansion efficiency in this prepared niche environment and finally determine the optimum seeding density, cells with different (high, medium, low) concentrations of 2×10⁶, 1×10⁶, 3×10⁵ cells /mL were separately seeded in three scaffold niches with the same elastic modulus (357 Pa) according to **(4)** and specific precursor ratios (dPG-BCN: pNIPAAm-co-PEG-N₃ = 2:3 according to **(3)**).

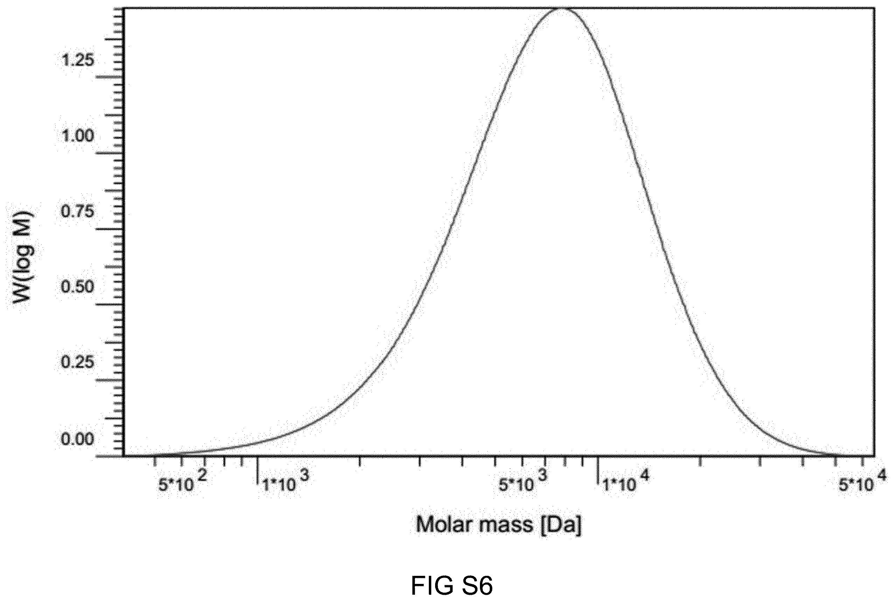

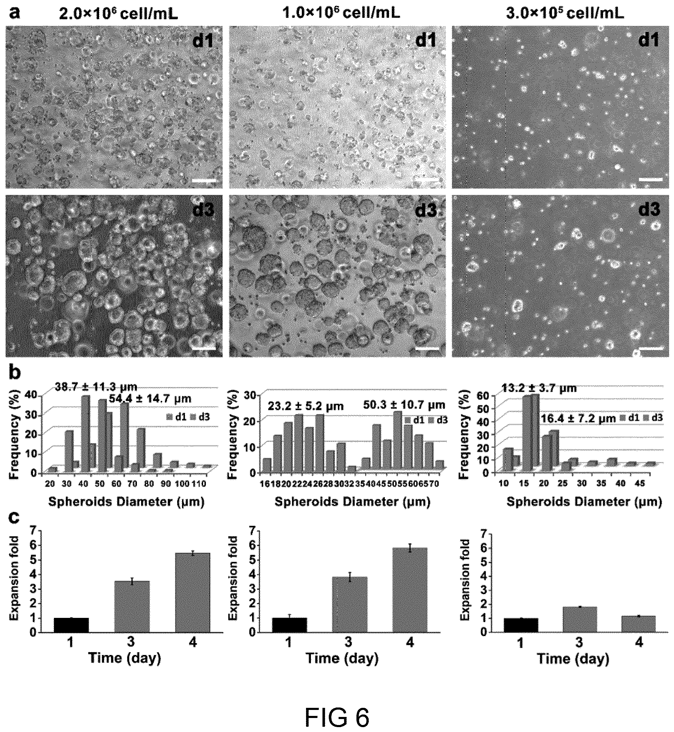

Figure 6 shows the optimization of the cell seeding density for iPSC expansion in dPG-pNIPAAm-co-PEG niche 3D culture system.

Figure 6a shows the morphology of the cells that survived in the artificial niche environment. Remarkably, under high or medium seeding density, the cells cultured grew very fast and formed EB soon, while, under low seeding density, the cells expanded and grew into spheroids slowly. According to the statistical analysis in Figure 6b, EB formed in the niche microenvironment with medium seeding density showed a much narrower size distribution than those under low and high seeding density conditions. Figure 6c shows that the expansion rates of iPSCs in niche with seeding density of 1×10⁶ cells /mL was 3.82 fold and 5.83 fold at day 3 and day 4, respectively, and was higher than those with seeding densities of 2×10⁶ cells /mL (3.54-fold and 5.47-fold at day 3 and day 4, respectively) and 3×10⁵ cells /mL (1.82-fold and 1.16-fold at day 3 and day 4, respectively). Overall, considering all the above factors, a seeding concentration of 1×10⁶ cells /mL can be chosen as the most reasonable seeding density for the iPSCs expansion in the described niche culture system.

### (6) Overall performance of the thermoresponsive hydrogel niche culture system in supporting iPSCs' proliferation

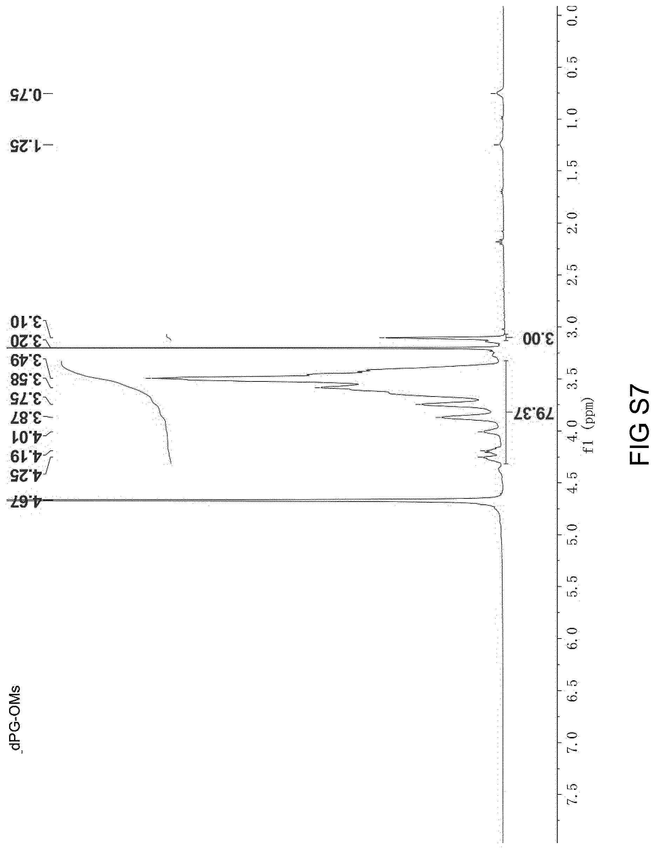

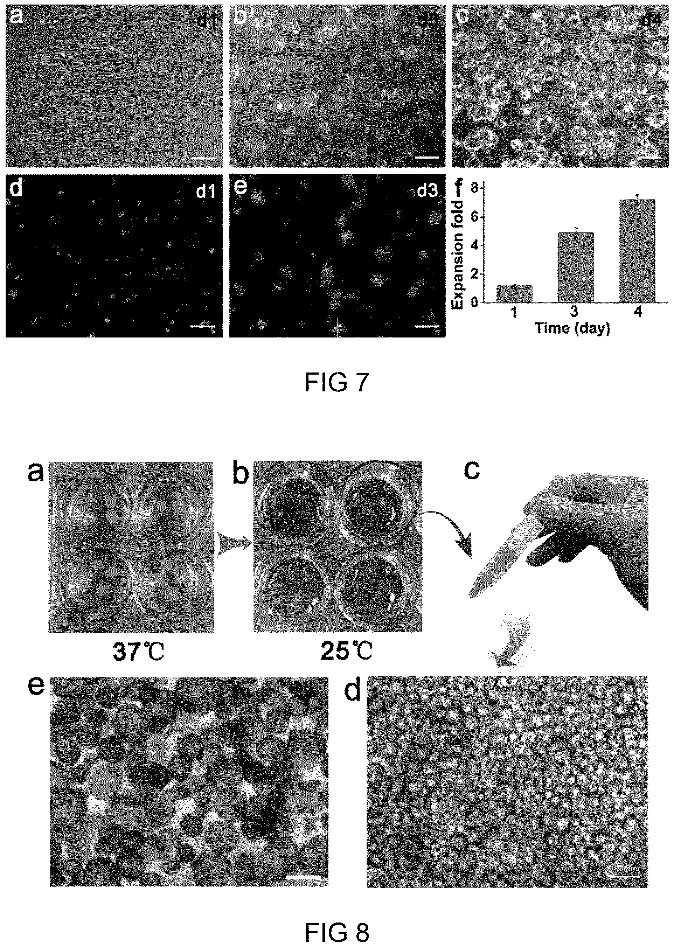

According to the above **(1)** - **(5)**, the inventors embedded iPSCs (1 × 10⁶ cells/mL) with precursors (dPG-BCN and pNIPAAm-co-PEG-N₃, both of their concentration were 100 mg/mL, the ratio of them was 2:3, cyclo (RGDfk) 1mM) into the culture system (E2~357 Pa), to investigate the cell viability and expansion efficiency and overall to assess the performance of the artificial 3D niche environment in iPSCs' culture (as shown in Figure 7).

Figure 7 shows that the dPG-pNIPAAm-co-PEG niche scaffold-based 3D culture system support iPSCs' expansion well under optimized conditions (scale bar = 100 µm).

The results in Figure 7 show that, under optimized conditions, single iPSCs cultured in the 3D microenvironment (with RGDSK culture medium) grew very fast and soon grew into dense, uniform, and small spheroids. Furthermore, the live/dead staining test indicated that cells cultured in this artificial environment had a high viability. Figure 7 a-c show these spheroids expanded quickly and took on narrow size distribution. Figure 7 d-e show that almost all cells kept their renewal ability. Together with Figure 7f, these results show that, under optimized conditions, the expansion rates of iPSCs in this niche culture system can achieve efficiency as high as 4.9- and 7.18-fold at day 3 and day 4, respectively. The study revealed that the dPG-pNIPAAm-co-PEG niche 3D culture system could allow for the robust proliferation of iPSCs with high viability. Overall, the cogelation of dPG-pNIPAAm-co-PEG-based culture system with optimized parameters could support iPSCs proliferation with excellent expansion efficiency without the limitation of a specific medium.

### (7) Controlled release and cell harvest

The core features of this thermoresponsive hydrogel based niche culture system presented in this invention is that they can be used to growth and controlled release of pluripotent stem cells. In particular, it is worth emphasizing that the proliferated cells could be controlled release out of the niches at any time under very mild conditions which has no harm to cells.

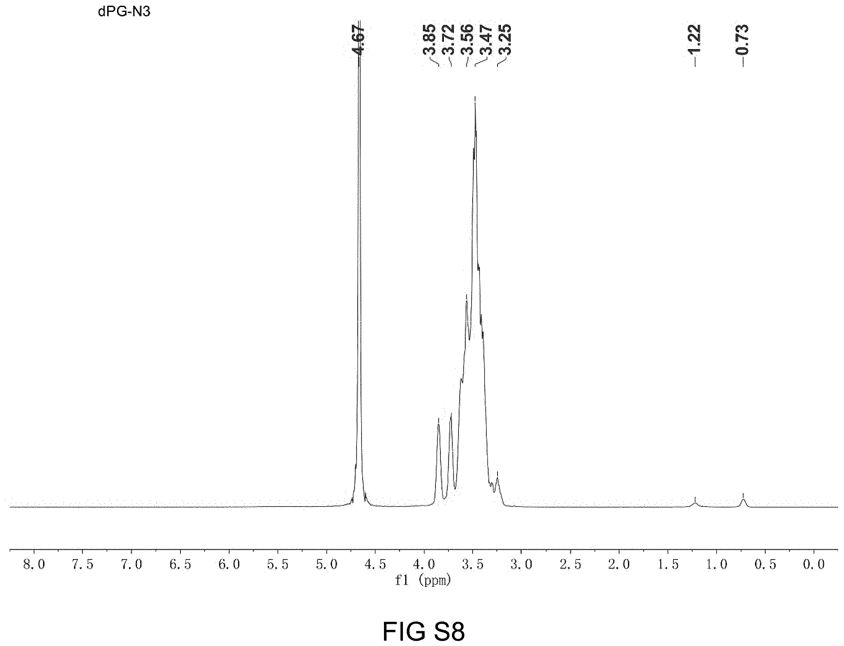

Figure 8 shows the controlled release and harvest of the expanded iPSCs from the dPG-pNIPAAm-co-PEG cogelation niche culture system.

Figure 8 shows that, by introducing a release property based on the mechanism of thermoreversible gelation into covalent crosslinking gelation and formed a physical and chemical cogelation system, the controlled release property of the thermoresponsive hydrogel was indeed achieved and this hydrogel niche culture system performed very well in controlled release and cell harvest.

In details, Figure 8a shows that after cultured for three days in cell incubator (37° C), the proliferated cells still survived very well. Figure 8a shows that, to release the expanded cells, the culture medium was replaced by the PBS, and the culture dish was incubated at 25° C (< LCST) for 5 min. During this process, due to the reversibility of the physical gelation process, the thermo-gelation disappeared, leaving behind a loose network of covalently crosslinked gel. Figure 8c shows that, after the morphology of the niche scaffold changed from white turbid solid to transparent and colorless, cells and niche scaffold were together transferred into a centrifuge tube. Figure 8d shows that by centrifugation, the released iPSCs' EB that formed in the niche environment were separated, collected, and finally achieved cell harvest under a very mild condition. Figure 8e shows that according to the preliminary proliferation test, continuous high expressions of alkaline phosphatase were detected during the culture period until the seventh day. This revealed that iPSCs' EB harvested from the culture system still maintained their proliferation ability well.

This study showed that the chemically defined dPG-pNIPAAm-co-PEG cogelation niche scaffold culture system not only offers many features that benefit iPSC cultures, such as supporting cells' rapid growth and prevention of forming large cell aggregates, but also supplies thermo-reversible degradation and thus enabling a controlled release of the expanded cells from their culture system. Moreover, the controlled release can be achieved by only adjusting the temperatures, which allows substantial convenience and maneuverability.

### (8) Cell quality characterization

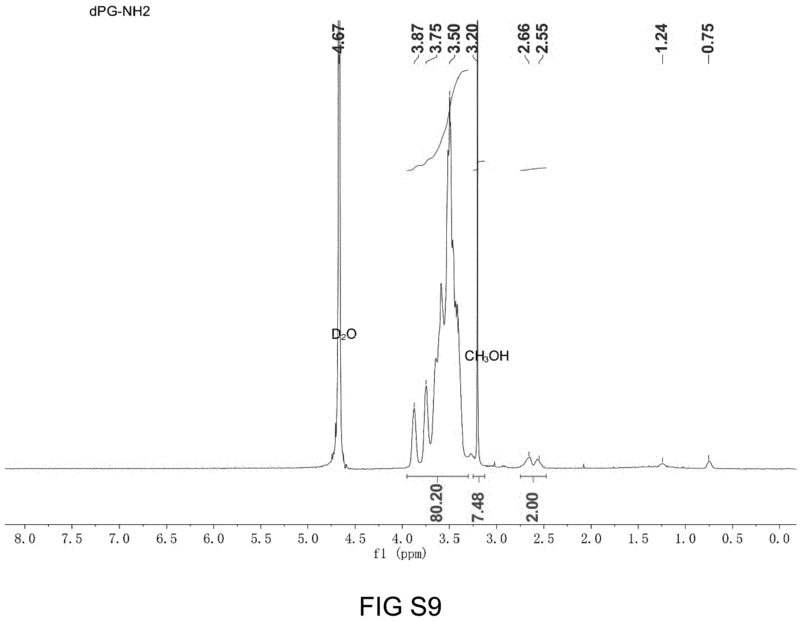

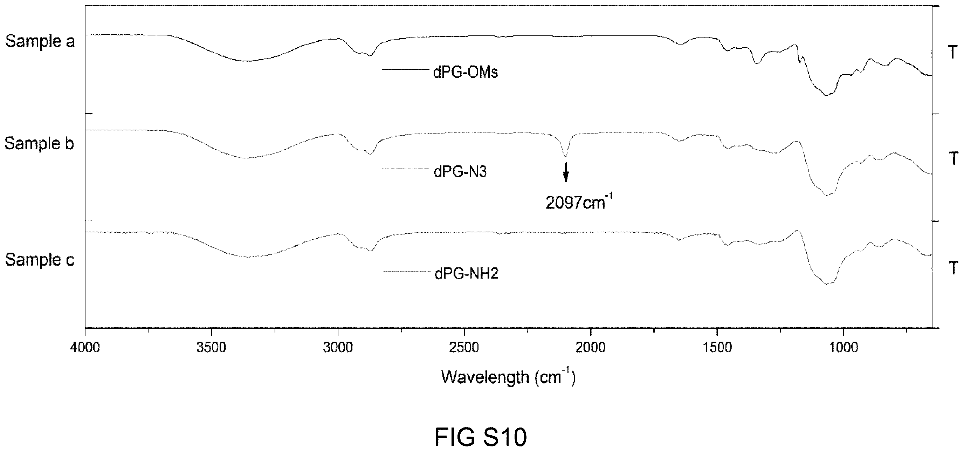

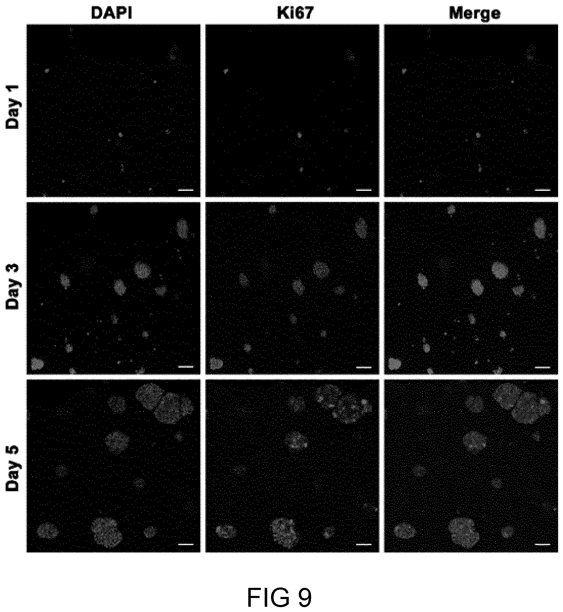

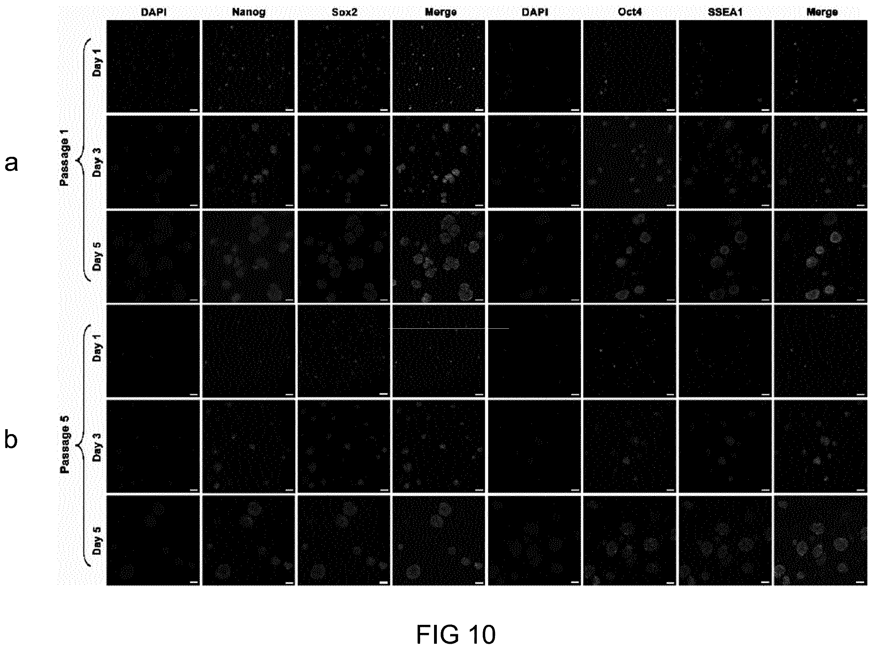

To further assess the performance of the chemically defined dPG-pNIPAAm-co-PEG cogelation niche scaffold culture system, Ki-67 staining test (Figure 9) and the immunocytochemistry analysis of pluripotency markers (Figure 10) were performed to investigate their proliferation ability and whether iPSCs could maintain pluripotency throughout the entire culturing process.

Firstly, investigations of Ki-67 staining from day 1 to day 5 in Figure 9 showed that iPSCs in the spheroids maintained a high expression of Ki-67 proliferation markers (in original coloring: blue: DAPI; red: a marker of proliferating cells). This means that the dPG-pNIPAAm-co-PEG cogelation niche scaffold culture system could support the expanded cells to keep high proliferation ability.

Beyond that, immune-fluorescent staining results shown in Figure 10a revealed that the chemically defined cogelation 3D niche-produced iPSCs in spheroids maintained high expression of pluripotent markers, including Nanog, Sox2, Oct4, and SSEA1. All the staining tests shown in Figure were performed after the iPSCs' EB were cultured for 1, 3, and 5 days, respectively (scale bar = 100 µm). In summary, together with the consistent cell growth and ALP staining in Figure 8d, the uniform expression of these proliferation and pluripotent markers deeply revealed that the current system highly expanded iPSCs with high quality, density, and efficiency.

Overall, all these Ki-67 staining, immunocytochemistry analysis and ALP staining measurements together demonstrated that this chemically defined, thermoresponsive, dPG-pNIPAAm-co-PEG cogelation microniche scaffold culture system supported iPSCs' proliferation and expansion well.

Typically, at least five passages are required to sufficiently assess pluripotency maintenance. Therefore, the cells that were harvested after three days culture (Figure 8d) were trypsin-dispersed into individual cells and cultured for another five passages. Then, the fifth passage cells were characterized by immunostaining the pluripotent markers of Nanog, Sox2, Oct4, and SSEA1 again after the cells had been cultured for one, three and five days separately (**Figure 10b**) (scale bar = 100 µm).The uniform expression of these pluripotent markers deeply revealed that the current system can still expand iPSCs with high quality performance at least even for 5 passages.

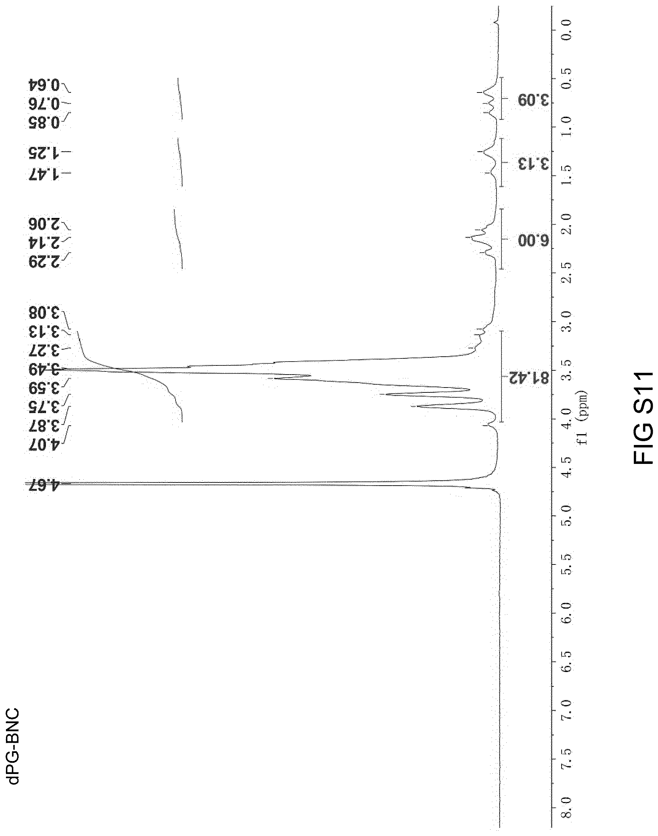

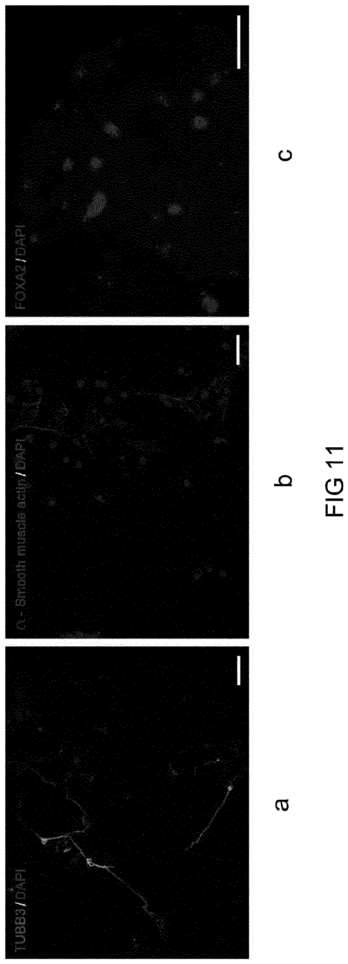

Additionally, to substantiate pluripotency maintenance in an alternative way, the present gold standard for investigating pluripotency maintenance, i.e., testing EB differentiation into the three germ layers, was applied. For this purpose, the cells were cultured over five passages. The cells from the fifth passage were characterized by the EB differentiation test. As shown in **Figure 11****,** immunostaining detected cells positive for α-smooth muscle actin (mesoderm marker) (Figure 11a), FOXA2 (endoderm marker) (Figure 11b), and β III tubulin (ectoderm marker) (Figure 11c). The scale bars in Figures 11a and 11b indicate 50 µm; the scale bar in Figure 11c indicates 100 µm. Secondary antibodies were labeled with Alexa 594 (red), except for TUBB3. Here, Alexa 488 (green) was used instead.

These results clearly indicate that, after having being cultured over five passages, the iPSCs could still differentiate into all three germ layers in vitro. These experiments demonstrate that the iPSCs produced by the presently described method indeed maintain their pluripotency.

### (9) Cell Culture

The mouse iPS cell line PhiC31 was obtained from System Biosciences (Catalog# SC211A-1). The iPSCs were usually maintained on plate coated with laminin (Cultrex, #3400-010-01) and cultured with complete clonal grade medium (Merck Millipore, #SF001- 500P) containing GSK3*β* inhibitor at 37 °C with 5% CO₂. The medium was usually changed daily and the cells were passaged every 3-4 days with Accutase (Merck Millipore, #SCR005). For iPSCs' 3D suspension culture, the cells were trypsinized with Accutase solution into single cells and then reseeded on uncoated plate with defined concentration. The medium was changed daily by gently centrifuging the cell spheroids into the bottom of the tubes and carefully aspirating the supernatant. For the 3D artificial niche culture, iPS cells were trypsinized with Accutase into single cells and then carefully aspirating the supernatant to get certain cell concentration before ready to use.

### (10) Controlled Release and Cell Harvest

After being cultured for certain time (at 37 °C with 5% CO₂), the cell expansion niche system could be transferred to room temperature (25 °C) conditions again for 5 min during which time the physical gelation disappeared and thus only chemical gelation was left in the niche system so that the niche was loosened, and expanded cells would release out of the niches. After centrifuging (400 r min⁻¹) for 5 min, the pure cells were harvested.

### (11) Spheroid Size Distribution

The cells in niche systems were imaged by normal or fluorescent microscope at specific time points and the cell spheroids' numbers and diameters were counted and measured by Imaged software.

### (12) Cell Viability

The viability of iPSCs in different culture system was measured by live/dead assay using a Live/Dead Viability kit (Thermo Fisher #L3224). Briefly, the live/dead dye solution was prepared by mixing 5 µL of 4 × 10⁻³ mol L⁻¹ calcein AM and 20 µL of 2 × 10⁻³ mol L⁻¹ EthD-1 stock solution into 10 mL of Dulbecco's phosphate-buffered saline. A freshly prepared staining solution was added to niche system with cells inside or cells finally got from PBS and then cells or spheroids were stained in the dark. After incubation for 40 min at 37 °C under a rocking device, the cells or spheroids were imaged by SP8-confocal microscope. Cell viability could be indicated by the green-fluorescent calcein-AM (indicated live cells) and the red-fluorescent ethidium homodimer-1 (indicated live cells), especially by their ratio in the live/ dead staining image. Besides the live/dead staining, cell viability could also be preliminarily indicated and monitored by the bright field microscope. Both of the cells in niche system or after released from the niche system could be monitored by their EB size distribution and frequency in different culture days, which could also be effective in indicating the cell viability and proliferation efficiency.

### (13) Cell Proliferation

Cell Counting Kit-8 (Sigma, #96992) was used to measure the expansion rate of the iPS cells growing in different culture systems. Briefly, at the beginning of an indicated time period, one tenth of the volume of CCK-8 reagent of the cell culture medium was added to each well of the plate with cells, and then incubated at 37 °C for 2 h. After incubation, the supernatants were transferred to 96-well plate, and the OD value for each well was read at wavelength 450 nm to determine the cell viability on a microplate reader. The assay was repeated thrice. The cell viability was calculated as following: cell viability (%) = (OD [experiment] - OD [blank])/(OD [control] - OD [blank] × 100).

### (14) Immunofluorescent Staining

After being released from the niche system at specified time periods, the cells were collected and washed with PBS (with 0.1% TritonX-100). Then the cells were fixed with 4% paraformaldehyde (PFA) (0.5% TritonX-100 in PBS) to fix them for 15-30 min, which was followed by permeabilization for 10-50 min (bigger cell spheroids need longer time). After washing with PBS for three times (putting on shaker rocking for 3 min each time), the cells were blocked with 1 mL 10% goat serum at 4 °C for overnight to cover nonspecific PBS with 0.1% TritonX-100 (anti-Nanog, 1:300, Abcam, #ab80892; anti-Sox2, 1:400, CST, #4900; anti-Oct4, 1:500, Abcam, #ab19857; anti- SSEA1, 1:300, Thermo Fisher, #MA1-022; anti-Ki-67, 1:400, CST, #9129) at 4 °C overnight. At least one blank sample was used as a negative control in these tests. Cells were washed again with PBS for three times the 2nd day and then incubated with secondary antibodies (goat anti-rabbit IgG H&L (Alexa Fluor 488), 1:400, Abcam, #ab150077; goat anti-mouse IgG H&L (Cy5), 1:1000, Abcam, #ab6563) under a dark environment at 37 °C for 1 h. After that, the cells were washed with PBS and stained with DAPI (1:2000) at room temperature for 30-40 min. Finally, after washing with PBS, the cells were imaged by sp8 confocal microscope.

### (15) Alkaline Phosphatase Staining

To confirm the pluripotency of iPSCs, ALP staining was performed by following the instructions of an ALP detection kit (Merck Millipore, #SCR004). Briefly, after collected and washed by PBS, cells spheroids were fixed by 4% PFA for very short time followed by PBS washing again. Then, they were transferred to the stain solution and then incubated at room temperature in dark for 15 min. After thrice washing with PBS, the images were observed and acquired by a color microscope.

### (16) In Vitro Differentiation of Induced Pluripotent Stem Cells

After 3 days' culture, cells were harvested and transferred onto gelatin-coated tissue culture dishes and also in the ESGRO medium without Gsk3*β* inhibitor and incubated for another 3 days. The cells were washed with PBS for 30 min and fixed with 4% PFA for 30 min at room temperature. Fixed cells were washed thrice with PBS and permeabilized in 0.5% Triton X-100 for 30 min at room temperature. Permeabilized cells were then blocked with 10% goat serum overnight at 4 °C. Cells were stained overnight with primary antibody at 4 °C. After three PBS washes, cells were stained with corresponding secondary antibody (1:1000; Invitrogen) overnight at 4 °C in the dark. Cells were washed thrice with PBS and incubated for 30 min in 4',6-diamidino-2-phenylindole (Invitrogen) for nuclei visualization. Confocal imaging was carried out using SP8 (Leica). The follows antibodies were used: *β* III tubulin (D71G9) Rabbit mAb (neuroectodermal marker, Cell signaling 5568T, 1:200), anti-smooth muscle actin (D4K9N) monoclonal Rabbit antibody (mesodermal marker, Cell signaling, 19245T, 1:200), and Rabbit anti- FOXA2 polyclonal antibody (Millipore, ref. no. AB4125, 1:200), goat anti-rabbit IgG H&L (Alexa Fluor 488, 1:1000, Abcam, #ab150077), and goat anti-rabbit IgG H&L (Alexa Fluor 594, Thermo Fisher, #A-11012, 1:1000)

### (17) Scanning Electron Microscopy Imaging

The niche gels formed by pNIPAAm-co-PEG azide and dPG-BCN were first put in a cell culture media overnight for swelling absolutely. After washing with PBS, the swollen niche gels were freeze-dried and then gold-sputtered for 30 s with gold deposition rate of 15 nm min⁻¹. SEM characterized the morphologies of niches.

### (18) Rheology

Rheological data were measured with a Malvern Instruments Kinexus Rheometer (with rSpace software) equipped with a parallel plate geometry and with an 8-mm plate, in particular with an 8-mm upper plate and a 65-mm lower plate (PU08:PL65). The plate gap was set to be 0.2 mm and the temperature was kept at 25 °C or 37 °C, respectively, for all experiments with an average normal force of ≈0.07 N. For the amplitude sweep, the scan range was set to 0.01-10% at 1 Hz. For frequency sweep, the frequency scan range was set to 0-10 Hz at 1%. All rheological experiments were repeated thrice.

In summary, according to the preceding sections **(1)** to **(18)**, embodiments of the present invention relate to a thermoresponsive hydrogel for the controlled release of iPSCs based on dPG and pNIPAAm-co-PEG polymers *via* physical-chemical-co-gelation. This thermoresponsive hydrogel supported the robust production of iPSCs with strong maintenance of pluripotency and self-renewal ability. Their expansion efficiency could reach as high as 4.9- and 7.2-folds within only in 3 and 4 days' culture, respectively. Due to the fully chemically defined properties, this 3D culture system could supply iPSCs without any reproduction limits and risks for pathogen and immunogenic transfer that might be caused by the application of traditional poorly defined animal-derived matrices or cell derivatives. Therefore, it exhibits a great promise for iPSCs to keep their full potential in downstream biomedical applications. Furthermore, the presented system obtained from the optimized physical-chemical-co-gelation strategy substantially overcomes the strong preference for defined medium and the collapse, which is caused by utilizing only thermoreversible physical gelation and would lead to being insufficient for preventing cells agglomeration. Most important of all, the introduction of thermoreversible crosslinkage to the covalently crosslinked networks equip the final 3D cogelation microniche culture system with specific thermoreversible degradation capability, which precisely enables the system to achieve a controlled release of the expanded cells by only changing the temperature in mild condition. This dramatically simplifies the process of cell harvesting. Additionally, compared with Matrigel or polypeptide based materials, the dendritic polyglycerol and poly(N-isopropylacrylamide) co-polyethylene glycol (pNIPAAm-co-PEG) polymers are really cheap, which would lead to a dramatic reduction in costs.

Overall, aspects of this invention provides an advanced, controlled-release, defined, artificial niche engineering approach based on the physical-chemical-co-gelation strategy that shows great promise in the field of iPSCs' 3D culture, and it would most likely has a strong market application prospect.

### List of references cited in the preceding sections:

1a K. Takahashi, S. Yamanaka, Cell, 2006, 126, 663-676.
1b R. Shrestha, Progress in Stem Cell, 2020, 7, 296-303.
1c K. Takahashi, S. Yamanaka, Development, 2013, 140, 2457-2461.
1d M. Scudellari, Nature, 2016, 534, 310-312.
2 M. X. Doss, A. Sachinidis, Cells, 2019, 8, 403.
3a M. Bellin, M. C. Marchetto, F. H. Gage, C. L. Mummery, Nat. Rev., 2012, 13, 713-726.
3b D. Doi, H. Magotani1, T. Kikuchi, M. Ikeda, S. Hiramatsu, K. Yoshida, N. Amano, M. Nomura, M. Umekage, A. Morizane, J. Takahashi, Nat. Commun., 2020, 11, 3369.
3c C. Yamashiro, K. Sasaki, S. Yokobayashi, Y. Kojima, M. Saitou, Nat. Protoc., 2020, 15, 1560-1583.
3d A. D. Baldassarre, E. Cimetta, S. Bollini, G. Gaggi, B. Ghinassi, Cells, 2018, 7, 48.
3e Y. Hayashi, M. Takami, M. M. Takasaki, Front. Cell. Neurosci., 2020, 4, 224.
3f R. N. Judson, M. V. Rossi, NPJ Regen. Med., 2020, 5, 10.
4a C.M. Fan, E. Zhang, J. Joshi, J.F. Yang, J.Y. Zhang, W.Q. Zhu, Front. Cell Dev. Biol., 2020, 8, 36.
4b M. Serra, C. Brito, C. Correia, P. M. Alves, Trends Biotechnol., 2012, 30, 350-359.
5 Y. Fan, J. Wu, P. Ashok, M. Hsiung, E. S. Tzanakakis, Stem Cell Rev., 2015, 11, 96-109.
6 H. Zhan, Dennis W. P. M. Löwik, Adv. Funct. Mater., 2019, 1808505.
7a I. Chimenti, D. Massai, U. Morbiducci, A. P. Beltrami, M. Pesce, E. Messina, J. Cardio. Transl. Res., 2017, 10, 150-166.
7b M. W. Lensch, L. Daheron, T. M. Schlaeger, Stem Cell Rev., 2006, 2, 185-201.
7c D. Jhala, R. Vasita, Polym. Rev., 2015, 55, 561-595.
8a A. J. Want, A. W. Nienow, C. J. Hewitt, K. Coopman, Regen. Med., 2012, 7, 71-84.
8b L. G. Villa-Diaz, A. M. Ross, J. Lahann, P. H. Krebsbach, Stem Cells, 2013, 31, 1-7.
8c L. Liu, K. I. Kamei, M. Yoshioka, M. Nakajima, J. Li, N. Fujimoto, S. Terada, Y. Tokunaga, Y. Koyama, H. Sato, K. Hasegawa, N. Nakatsuji, Y. Chen, Biomaterials, 2017, 124, 47-54.
9 F. C. Paccola Mesquita, C. Hochman-Mendez, J. Morrissey, L. C. Sampaio, D. A. Taylor, Stem Cells Int., 2019, 9704945.
10 J. Thiele, Y. Ma, S. M. Bruekers, S. Ma, W. T. Huck, Adv. Mater., 2014, 26, 125-147.
11 M. Bao, J. Xie, W. T. S. Huck, Adv. Sci., 2018, 5, 1800448.
12 K. G. Chen, B. S. Mallon, R. D. McKay, P. G. Robey, Cell Stem Cell, 2014, 14, 13-26.
13a R. Cruz-Acuna, M. Quiros, A. E. Farkas, P. H. Dedhia, S. Huang, D. Siuda, V. Garcia-Hernandez, A. J. Miller, J. R. Spence, A. Nusrat, A. J. Garcia, Nat. Cell Biol., 2017, 19, 1326-1335.
13b K. R. Stevens, C. E. Murry, Cell Stem Cell, 2018, 22, 294-297.
13c S. P. Pasca, Nature, 2018, 553, 437-445.
13d N. Gjorevski, N. Sachs, A. Manfrin, S. Giger, M. E. Bragina, P. Ordonez-Moran, H. Clevers, M. P. Lutolf, Nature, 2016, 539, 560-564.
14a M. P. Lutolf, P. M. Gilbert, H. M. Blau, Nature, 2009, 462, 433-441.
14b A. M. Akimoto, E. H. Niitsu, K. Nagase, T. Okano, H. Kanazawa, R. Yoshida, Int. J. Mol. Sci., 2018, 19, 1253.
14c T. C. Sunga, H.F. Li, A. Higuchi, S. S. Kumarg, Q. D. Lingh, Y.W. Wui, T. Burnouf, M. Nasud, A. Umezawad, K.F. Leel, H. C. Wangm, Y. Change, S. T. Hsun, Biomaterials, 2020, 230, 119638.
14d S. M. Vornholt, R. E. Morris, Nat. Mater., 2019, 18, 910-911.
15a I. C. Yasa, A. F. Tabak, O. Yasa, H. Ceylan, M. Sitti, Adv. Funct. Mater., 2019, 29, 1808992.
15b J. Koh, D. R. Griffin, M. M. Archang, A. C. Feng, T. Horn, M. Margolis, D. Zalazar, T. Segura, P. O. Scumpia, D. Di Carlo, Small, 2019, e1903147.
15c R.Y. Chen, L. Li, L. Feng, Y.X. Luo, M.E. Xu, K. W. Leong, R. Yao, Biomaterials, 2020, 230, 119627.
16a D. Seliktar, Science, 2012, 336, 1124-1128.
16b E. Prince, E. Kumacheva, Nat. Rev. Mater., 2019, 4, 99-115.
16c M. Caiazzo, Y. Okawa, A. Ranga, A. Piersigilli, Y. Tabata, M. P. Lutolf, Nat. Mater., 2016, 15, 344-352.
17 F. M. Chen, X. Liu, Prog. Polym. Sci., 2016, 53, 86-168.
18 D. Steinhilber, T. Rossow, S. Wedepohl, F. Paulus, S. Seiffert, R. Haag, Angew. Chem. Int. Ed., 2013, 52, 13538-13543.
19 Y. Lei, D. V. Schaffer, PNAS, 2013, 110, E5039-5048.
20 B.L. Seala, T.C. Oterob, A. Panitcha, Mater. Sci. Eng. R Rep., 2001, 34, 147-230.
21a P. Zhou, B. Yin, R. Zhang, Z. Xu, Y. Liu, Y. Yan, X. Zhang, S. Zhang, Y. Li, H. Liu, Y. A. Yuan, S. Wei, Colloids Surf. B, 2018, 171, 451-460.
21b I. Lilge, H. Schonherr, Angew. Chem. Int. Ed., 2016, 55, 13114-13117.
22 T. Scholzen, J. Gerdes, J. cell. physiol., 2000, 182, 311-322.
23 J. Tu, G. Tian, H. H. Cheung, W. Wei, T. L. Lee, Stem Cell Res. Ther., 2018, 9, 71.
24 Y. Zhou, H. Mao, B. Joddar, N. Umeki, Y. Sako, K.-l. Wada, C. Nishioka, E. Takahashi, Y. Wang, Y. Ito, Sci. Rep., 2015, 5, 11386.
25 D. Thomas, T. O'Brien, A. Pandit, Adv. Mater., 2018, 30, 1703948.
26 K. H. Vining, A. Stafford, D. J. Mooney, Biomaterials, 2019, 188, 187-197.
27 S. Talebian, M. Mehrali, N. Taebnia, C. P. Pennisi, F. B. Kadumudi, J. Foroughi, M. Hasany, M. Nikkhah, M. Akbari, G. Orive, A. D. Pirouz, Adv. Sci., 2019, 6, 1801664.
28 M. Sofman, A. Brown, L. G. Griffith, P. T. Hammond, Biomaterials, 2020, 120231.
29a H. G. Schild, Prog. Polym. Sci., 1992, 17, 163-249.
29b T. Sun, G. Qing, Adv. Mater., 2011, 23, H57-H77.
30 B. Trappmann, J. E. Gautrot, J. T. Connelly, D. G. Strange, Y. Li, M. L. Oyen, M. A. Cohen Stuart, H. Boehm, B. Li, V. Vogel, J. P. Spatz, F. M. Watt, W. T. Huck, Nat. Mater., 2012, 11, 642-649.
31 S. Bhatia, D. Lauster, M. Bardua, K. Ludwig, S. Angioletti-Uberti, N. Popp, U. Hoffmann, F. Paulus, M. Budt, M. Stadtmuller, T. Wolff, A. Hamann, C. Bottcher, A. Herrmann, R. Haag, Biomaterials 2017, 138, 22-34.
32a S. B. Anderson, C. C. Lin, D. V. Kuntzler, K. S. Anseth, Biomaterials 2011, 32, 3564-3574
32b F. Stijn, M. Dongen, Adv. Mater. 2013, 25, 1687-1691.
33 V. Cheng, B. H. Lee, C. Pauken, B. L. Vernon, J. Appl. Polym. Sci., 2007, 106, 1201-1207.
34 S. Roller, H. Zhou, R. Haag, Mol. Divers. 2005, 9, 305-316.

## Claims

1. Thermoresponsive hydrogel, comprising dendritic polyglycerol units and poly(N-isopropylacrylamide)-co-polyethylene glycol units that are covalently bound to each other via a linker, wherein the linker is chosen from the group consisting of a cyclic triazol moiety and a diazacyclohexadiene moiety.

2. Thermoresponsive hydrogel according to claim 1, further comprising at least one of a cyclo(-RGDfK) peptide and a cyclo(-RGDfk) peptide covalently bound to one of the dendritic polyglycerol units or the poly(N-isopropylacrylamide)-co-polyethylene glycol units.

3. Thermoresponsive hydrogel according to claim 1 or 2, **characterized in that** a molar ratio between N-isopropylacrylamide and poly(ethylene glycol) in the poly(N-isopropylacrylamide)-co-polyethylene glycol units lies in a range of from 30:1 to 1:1.

4. Thermoresponsive hydrogel according to any of the preceding claims, **characterized in that** a molar ratio between the dendritic polyglycerol units and the poly(N-isopropylacrylamide)-co-polyethylene glycol units lies in a range of from 1:3 to 1:1.

5. Thermoresponsive hydrogel according to any of the preceding claims, **characterized in that** the thermoresponsive hydrogel has an elastic modulus G', measured at 25 °C or at 37 °C with an 8-mm plate, lying in a range of from 15 Pa to 1500 Pa.

6. Use of a thermoresponsive hydrogel according to any of the preceding claims for cultivating cells in vitro.

7. Use according to claim 6, **characterized in that** the cells are induced pluripotent stem cells.

8. Method for cultivating cells in vitro, comprising the following steps:
a) mixing cells to be cultivated with i) a thermoresponsive hydrogel according to any of claims 1 to 5 or ii) a dendritic polyglycerol polymer derivative and a poly(N-isopropylacrylamide)-co-polyethylene glycol derivative that spontaneously form a thermoresponsive hydrogel according to any of claims 1 to 5, to obtain a mixture of the thermoresponsive hydrogel and the cells to be cultivated, wherein the mixing is carried out at a first temperature, the first temperature lying below a transition temperature of the thermoresponsive hydrogel, the transition temperature of the thermoresponsive hydrogel lying in a range of from 29 °C to 35 °C;
b) incubating the mixture of the thermoresponsive hydrogel and the cells to be cultivated at the first temperature for a first period of time;
c) increasing the temperature of the mixture of the thermoresponsive hydrogel and the cells to be cultivated to a second temperature, the second temperature lying above the transition temperature of the thermoresponsive hydrogel;
d) cultivating the cells to be cultivated at the second temperature for a second period of time to obtain a mixture of the thermoresponsive hydrogel and cultivated cells;
e) decreasing the temperature of the mixture of the thermoresponsive hydrogel and the cultivated cells to a third temperature, the third temperature lying below the transition temperature of the thermoresponsive hydrogel;
f) separating the cultivated cells from the mixture of the thermoresponsive hydrogel and the cultivated cells at the third temperature;
g) harvesting the cultivated cells.

9. Method according to claim 8, **characterized in that** the cells to be cultivated are induced pluripotent stem cells.

10. Method according to claim 8 or 9, **characterized in that** the dendritic polyglycerol polymer derivative is dendritic polyglycerol bicyclononyne and/or **in that** the poly(N-isopropylacrylamide)-co-polyethylene glycol derivative is poly(N-isopropylacrylamide)-co-polyethylene glycol azide.

11. Method according to any of claims 8 to 10, **characterized in that** the third temperature corresponds to the first temperature.

12. Method according to any of claims 8 to 11, **characterized in that** the first temperature and/or the third temperature lie in a range of from 10 °C to 26 °C, and/or **in that** the second temperature lies in a range of from 36 °C to 38 °C.

13. Method according to any of claims 8 to 12, **characterized in that** the thermoresponsive hydrogel in the mixture of the thermoresponsive hydrogel and the cells to be cultivated further comprises at least one of a cyclo(-RGDfK) peptide and a cyclo(-RGDfk) peptide covalently bound to one of the dendritic polyglycerol units or the poly(N-isopropylacrylamide)-co-polyethylene glycol units of the thermoresponsive hydrogel.

14. Method according to any of claims 8 to 13, **characterized in that** the separating of the cultivated cells is carried out by centrifuging the mixture of the thermoresponsive hydrogel and the cultivated cells.

15. Method for manufacturing a thermoresponsive hydrogel according to any of claims 1 to 5, comprising the following step:
a) mixing a dendritic polyglycerol polymer derivative and a poly(N-isopropylacrylamide)-co-polyethylene glycol derivative that spontaneously form a thermoresponsive hydrogel according to any of claims 1 to 6, wherein the mixing is carried out at a first temperature, the first temperature lying below a transition temperature of the thermoresponsive hydrogel, the transition temperature of the thermoresponsive hydrogel lying in a range of from 29 °C to 35 °C.

## Patentansprüche

1. Thermoresponsives Hydrogel, umfassend Einheiten von dendritischem Polyglycerin und Poly(N-isopropylacrylamid)-co-Polyethylenglykol-Einheiten, die über einen Linker kovalent miteinander verbunden sind, wobei der Linker ausgewählt ist aus der Gruppe bestehend aus einer cyclischen Triazol-Einheit und einer Diazacyclohexadien-Einheit.

2. Thermoresponsives Hydrogel nach Anspruch 1, weiterhin umfassend mindestens eines eines Cyclo(-RGDfK)-Peptids und einesCyclo(-RGDfk)-Peptids, das kovalent an eines von den Einheiten von dendritischem Polyglycerin oder den Poly(N-isopropylacrylamid)-co-Polyethylenglykol-Einheiten gebunden ist.

3. Thermoresponsives Hydrogel nach Anspruch 1 oder 2, **dadurch gekennzeichnet, dass** ein Molverhältnis zwischen N-Isopropylacrylamid und Polyethylenglykol in den Poly(N-isopropylacrylamid)-co-Polyethylenglykol-Einheiten in einem Bereich von 30:1 bis 1:1 liegt.

4. Thermoresponsives Hydrogel nach einem der vorangehenden Ansprüche, **dadurch gekennzeichnet, dass** ein Molverhältnis zwischen den Einheiten von dendritischem Polyglycerin und den Poly(N-isopropylacrylamid)-co-Polyethylenglykol-Einheiten in einem Bereich von 1:3 bis 1:1 liegt.

5. Thermoresponsives Hydrogel nach einem der vorangehenden Ansprüche, **dadurch gekennzeichnet, dass** das thermoresponsive Hydrogel einen Elastizitätsmodul G', gemessen bei 25 °C oder bei 37 °C mit einer 8-mm-Platte, aufweist, der in einem Bereich von 15 Pa bis 1500 Pa liegt.

6. Verwendung eines thermoresponsiven Hydrogels nach einem der vorangehenden Ansprüche zum Kultivieren von Zellen in vitro.

7. Verwendung nach Anspruch 6, **dadurch gekennzeichnet, dass** die Zellen induzierte pluripotente Stammzellen sind.

8. Verfahren zum Kultivieren von Zellen in vitro, umfassend die folgenden Schritte:
a) Mischen von zu kultivierenden Zellen mit i) einem thermoresponsiven Hydrogel nach einem der Ansprüche 1 bis 5 oder ii) einem Polymerderivat von dendritischem Polyglycerin und einem Derivat von Poly(N-isopropylacrylamid)-co-Polyethylenglykol, die spontan ein thermoresponsives Hydrogel nach einem der Ansprüche 1 bis 5 bilden, um ein Gemisch aus dem thermoresponsiven Hydrogel und den zu kultivierenden Zellen zu erhalten, wobei das Mischen bei einer ersten Temperatur durchgeführt wird, wobei die erste Temperatur unterhalb einer Übergangstemperatur des thermoresponsiven Hydrogels liegt, wobei die Übergangstemperatur des thermoresponsiven Hydrogels in einem Bereich von 29 °C bis 35 °C liegt;
b) Inkubieren des Gemisches aus dem thermoresponsiven Hydrogel und den zu kultivierenden Zellen bei der ersten Temperatur über eine erste Zeitspanne;
c) Erhöhen der Temperatur des Gemisches aus dem thermoresponsiven Hydrogel und den zu kultivierenden Zellen auf eine zweite Temperatur, wobei die zweite Temperatur oberhalb der Übergangstemperatur des thermoresponsiven Hydrogels liegt;
d) Kultivieren der zu kultivierenden Zellen bei der zweiten Temperatur über eine zweite Zeitspanne, um ein Gemisch aus dem thermoresponsiven Hydrogel und kultivierten Zellen zu erhalten;
e) Verringern der Temperatur des Gemisches aus dem thermoresponsiven Hydrogel und den kultivierten Zellen auf eine dritte Temperatur, wobei die dritte Temperatur unterhalb der Übergangstemperatur des thermoresponsiven Hydrogels liegt;
f) Abtrennen der kultivierten Zellen aus dem Gemisch aus dem thermoresponsiven Hydrogel und den kultivierten Zellen bei der dritten Temperatur;
g) Ernten der kultivierten Zellen.

9. Verfahren nach Anspruch 8, **dadurch gekennzeichnet, dass** die zu kultivierenden Zellen induzierte pluripotente Stammzellen sind.

10. Verfahren nach Anspruch 8 oder 9, **dadurch gekennzeichnet, dass** das Polymerderivat von dendritischem Polyglycerin (dendritisches Polyglycerin)-Bicyclononin ist und/oder dadurch, dass das Derivat von Poly(N-isopropylacrylamid)-co-Polyethylenglykol Poly(N-isopropylacrylamid)-co-Polyethylenglykolazid ist.

11. Verfahren nach einem der Ansprüche 8 bis 10, **dadurch gekennzeichnet, dass** die dritte Temperatur der ersten Temperatur entspricht.

12. Verfahren nach einem der Ansprüche 8 bis 11, **dadurch gekennzeichnet, dass** die erste Temperatur und/oder die dritte Temperatur in einem Bereich von 10 °C bis 26 °C liegen, und/oder dadurch, dass die zweite Temperatur in einem Bereich von 36 °C bis 38 °C liegt.

13. Verfahren nach einem der Ansprüche 8 bis 12, **dadurch gekennzeichnet, dass** das thermoresponsive Hydrogel in dem Gemisch aus dem thermoresponsiven Hydrogel und den zu kultivierenden Zellen weiterhin mindestens eines eines Cyclo(-RGDfK)-Peptids und eines Cyclo(-RGDfk)-Peptids, das kovalent an eines von den Einheiten von dendritischem Polyglycerin oder den Poly(N-isopropylacrylamid)-co-Polyethylenglykol-Einheiten des thermoresponsiven Hydrogels gebunden ist, umfasst.

14. Verfahren nach einem der Ansprüche 8 bis 13, **dadurch gekennzeichnet, dass** das Abtrennen der kultivierten Zellen durch Zentrifugieren des Gemisches aus dem thermoresponsiven Hydrogel und den kultivierten Zellen durchgeführt wird.

15. Verfahren zur Herstellung eines thermoresponsiven Hydrogels nach einem der Ansprüche 1 bis 5, umfassend den folgenden Schritt:
a) Mischen eines Polymerderivates von dendritischem Polyglycerin und eines Derivates von Poly(N-isopropylacrylamid)-co-Polyethylenglykol, die spontan ein thermoresponsives Hydrogel nach einem der Ansprüche 1 bis 6 bilden, wobei das Mischen bei einer ersten Temperatur durchgeführt wird, wobei die erste Temperatur unterhalb einer Übergangstemperatur des thermoresponsiven Hydrogels liegt, wobei die Übergangstemperatur des thermoresponsiven Hydrogels in einem Bereich von 29 °C bis 35 °C liegt.

## Revendications

1. Hydrogel thermosensible comprenant des unités de polyglycérol dendritique et des unités de poly(N-isopropylacrylamide)-co-polyéthylène glycol qui sont liées de manière covalente les unes aux autres par l'intermédiaire d'un lieur, dans lequel le lieur est choisi parmi le groupe constitué par une fraction triazol cyclique et une fraction diazacyclohexadiène.

2. Hydrogel thermosensible selon la revendication 1, comprenant en outre au moins un d'un peptide cyclo(-RGDfK) et d'un peptide cyclo(-RGDfk) lié de manière covalente à une des unités de polyglycérol dendritique ou des unités de poly(N-isopropylacrylamide)-co-polyéthylène glycol.

3. Hydrogel thermosensible selon la revendication 1 ou 2, **caractérisé en ce qu'**un rapport molaire entre le N-isopropylacrylamide et le poly(éthylène glycol) dans les unités de poly(N-isopropylacrylamide)-co-polyéthylène glycol se situe dans une plage allant de 30:1 à 1:1.

4. Hydrogel thermosensible selon l'une quelconque des revendications précédentes, **caractérisé en ce qu'**un rapport molaire entre les unités de polyglycérol dendritique et les unités de poly(N-isopropylacrylamide)-co-polyéthylène glycol se situe dans une plage allant de 1:3 à 1:1.

5. Hydrogel thermosensible selon l'une quelconque des revendications précédentes, **caractérisé en ce que** l'hydrogel thermosensible présente un module élastique G', mesuré à 25 °C ou à 37 °C avec une plaque de 8 mm, situé dans une plage allant de 15 Pa à 1500 Pa.

6. Utilisation d'un hydrogel thermosensible selon l'une quelconque des revendications précédentes pour la culture de cellules in vitro.

7. Utilisation selon la revendication 6, **caractérisée en ce que** les cellules sont des cellules souches pluripotentes induites.

8. Procédé de culture de cellules in vitro, comprenant les étapes suivantes :
a) le mélange de cellules à cultiver avec i) un hydrogel thermosensible selon l'une quelconque des revendications 1 à 5 ou ii) un dérivé polymère de polyglycérol dendritique et un dérivé de poly(N-isopropylacrylamide)-co-polyéthylène glycol qui forment spontanément un hydrogel thermosensible selon l'une quelconque des revendications 1 à 5, pour obtenir un mélange de l'hydrogel thermosensible et des cellules à cultiver, dans lequel le mélange est réalisé à une première température, la première température étant située sous une température de transition de l'hydrogel thermosensible, la température de transition de l'hydrogel thermosensible étant située dans une plage allant de 29 °C à 35 °C ;
b) l'incubation du mélange de l'hydrogel thermosensible et des cellules à cultiver à la première température pendant une première période de temps ;
c) l'augmentation de la température du mélange de l'hydrogel thermosensible et des cellules à cultiver à une deuxième température, la deuxième température étant située au-dessus de la température de transition de l'hydrogel thermosensible ;
d) la culture des cellules à cultiver à la deuxième température pendant une deuxième période de temps pour obtenir un mélange de l'hydrogel thermosensible et des cellules cultivées ;
e) la diminution de la température du mélange de l'hydrogel thermosensible et des cellules cultivées jusqu'à une troisième température, la troisième température étant située sous la température de transition de l'hydrogel thermosensible ;
f) la séparation des cellules cultivées du mélange de l'hydrogel thermosensible et des cellules cultivées à la troisième température ;
g) la récolte des cellules cultivées.

9. Procédé selon la revendication 8, **caractérisé en ce que** les cellules à cultiver sont des cellules souches pluripotentes induites.

10. Procédé selon la revendication 8 ou 9, **caractérisé en ce que** le dérivé polymère de polyglycérol dendritique est un bicyclononyne de polyglycérol dendritique et/ou **en ce que** le dérivé de poly(N-isopropylacrylamide)-co-polyéthylène glycol est un azide de poly(N-isopropylacrylamide)-co-polyéthylène glycol.

11. Procédé selon l'une quelconque des revendications 8 à 10, **caractérisé en ce que** la troisième température correspond à la première température.

12. Procédé selon l'une quelconque des revendications 8 à 11, **caractérisé en ce que** la première température et/ou la troisième température se situent dans une plage allant de 10 °C à 26 °C, et/ou **en ce que** la deuxième température se situe dans une plage allant de 36 °C à 38 °C.

13. Procédé selon l'une quelconque des revendications 8 à 12, **caractérisé en ce que** l'hydrogel thermosensible dans le mélange de l'hydrogel thermosensible et des cellules à cultiver comprend en outre au moins un d'un peptide cyclo(-RGDfK) et d'un peptide cyclo(-RGDfk) liés à une des unités de polyglycérol dendritique et des unités de poly(N-isopropylacrylamide)-co-polyéthylène glycol de l'hydrogel thermosensible.

14. Procédé selon l'une quelconque des revendications 8 à 13, **caractérisé en ce que** la séparation des cellules cultivées est réalisée par la centrifugation du mélange de l'hydrogel thermosensible et des cellules cultivées.

15. Procédé de préparation d'un hydrogel thermosensible selon l'une quelconque des revendications 1 à 5, comprenant l'étape suivante :
a) le mélange d'un dérivé polymère de polyglycérol dendritique et d'un dérivé de poly(N-isopropylacrylamide)-co-polyéthylène glycol qui forment spontanément un hydrogel thermosensible selon l'une quelconque des revendications 1 à 6, dans lequel le mélange est réalisé à une première température, la première température étant située sous une température de transition de l'hydrogel thermosensible, la température de transition de l'hydrogel thermosensible étant située dans une plage allant de 29 °C à 35 °C.
